# EUROPÄISCHE PATENTSCHRIFT

(11) **EP 3 066 065 B1**
(45) Veröffentlichungstag und Bekanntmachung des Hinweises auf die Patenterteilung: **19.06.2019**
(21) Anmeldenummer: 14787135.4
(22) Anmeldetag: 16.10.2014
(51) Int. Cl.: C07B 43/04, C07C 209/50, C07C 227/16, C07D 265/32, C07D 207/16, C07D 295/03, C07D 295/15, C07F 9/40, C07K 5/00

(54) **KATALYTISCHE HYDRIERUNG ZUR HERSTELLUNG VON AMINEN AUS CARBONSÄUREAMIDEN, CARBONSÄUREDIAMIDEN, DI-, TRI- ODER POLYPEPTIDEN ODER PEPTIDAMIDEN**
CATALYTIC HYDROGENATION FOR THE PREPARATION OF AMINES FROM CARBOXYLIC ACID AMIDES, CARBOXYLIC ACID AMIDES, DI-, TRI- OR POLYPEPTIDES OR PEPTIDE AMIDES
HYDROGÉNISATION CATALYTIQUE POUR LA FABRICATION D'AMINES À PARTIR D'AMIDES D'ACIDE CARBONIQUE, DE DIAMIDES D'ACIDE CARBONIQUE, DE DI-, DE TRI- OU DE POLYPEPTIDES OU DE PEPTIDAMIDES

(30) Priorität: 05.11.2013 EP 13191503
(43) Veröffentlichungstag der Anmeldung: 14.09.2016
(73) Patentinhaber: Evonik Degussa GmbH, 45128 Essen (DE)
(72) Erfinder: KADYROV, Renat, 60389 Frankfurt (DE)
(74) Vertreter: Evonik Patent Association
(86) Internationale Anmeldenummer: PCT/EP2014/072184
(87) Internationale Veröffentlichungsnummer: WO 2015/067450

(56) Entgegenhaltungen:
- US-A1- 2012 253 042
- MARIO STEIN ET AL: "Catalytic Hydrogenation of Amides to Amines under Mild Conditions", ANGEWANDTE CHEMIE INTERNATIONAL EDITION, Bd. 52, Nr. 8, 18. Februar 2013 (2013-02-18), Seiten 2231-2234, XP055107456, ISSN: 1433-7851, DOI: 10.1002/anie.201207803 in der Anmeldung erwähnt
- EKAMBARAM BALARAMAN ET AL: "Direct Hydrogenation of Amides to Alcohols and Amines under Mild Conditions", JOURNAL OF THE AMERICAN CHEMICAL SOCIETY, Bd. 132, Nr. 47, 1. Dezember 2010 (2010-12-01), Seiten 16756-16758, XP055016966, ISSN: 0002-7863, DOI: 10.1021/ja1080019

## Beschreibung

Die vorliegende Erfindung betrifft ein Verfahren zur Herstellung von Aminen, umfassend die folgenden Schritte: a. Umsetzung eines (i) Carbonsäureamids der allgemeinen Formel (I), oder (ii) Carbonsäurediamids der allgemeinen Formel (II), oder (iii) Di-, Tri- oder Polypeptids, oder (iv) Peptidamids mit carboxyterminaler Amidfunktion mit einem Alkylierungsmittel, b. Zugabe eines Hydrierkatalysators, der mindestens ein aktives Metall der Gruppe VII B und/oder VIII B des Periodensystems der Elemente enthält, zum Reaktionsgemisch in einem Mol-Verhältnis von 1:10 bis 1:100.000 bezogen auf Carbonsäureamid, Carbonsäurediamid, Di-, Tri- oder Polypeptid oder Peptidamid, c. Umsetzung des Reaktionsgemisches mit Wasserstoff, wobei ein Wasserstoffdruck von 0,1 bar bis 200 bar eingestellt wird und wobei eine Temperatur in einem Bereich von 0°C bis 120°C eingestellt wird.

Die Reduktion von Carbonsäureamiden gehört mit zu den wichtigsten Methoden für die Herstellung von Aminen. Das klassische Verfahren basiert auf der Reduktion durch komplexe Hydride, wobei jedoch stöchiometrische Mengen an Hydrid erforderlich sind und die Selektivität relativ gering ist. Die Entwicklung der katalytischen Reduktion mit Wasserstoff bleibt bis heute eine der größten Herausforderungen. In der Literatur sind derartige Hydrierungen bekannt, jedoch sind große Mengen (15 mol% und mehr) an Katalysator, sehr hohe Drücke und Temperaturen über 200°C notwendig, um brauchbare Ausbeuten zu erzielen (S. Nishimura, Handbook of Heterogeneous Catalytic Hydrogenation for Organic Synthesis 2001, pp. 406-411, Wiley, N.Y.). Kürzlich wurde über die Hydrierung von tertiären und sekundären Carbonsäureamiden zu Aminen bei 120-160°C über einen bimetallischen Pd-Re-Katalysator berichten (M. Stein , B. Breit, Angew. Chem. 2013, 125, 2287-2290). Trotz etwas milderer Bedingungen wird jedoch kaum eine funktionelle Gruppe toleriert, sogar olefinische Doppelbindungen und aromatische Ringe werden durchhydriert.

Die Aufgabe der vorliegenden Erfindung ist es daher, ein verbessertes Verfahren für die katalytische Hydrierung von Carbonsäureamiden mittels Wasserstoff zur Verfügung zu stellen, das nicht die Nachteile der bisher verwendeten direkten Hydrierung aufweist. Vielmehr sollte das Augenmerk auf die Milde der Reaktionsbedingungen und große Toleranz gegenüber verschiedensten funktionellen Gruppen gerichtet sein.

Überraschenderweise wurde nun gefunden, dass O-alkylierte Carbonsäureamide unter sehr milden Bedingungen in Gegenwart von üblichen Hydrierkatalysatoren zu Aminen hydriert werden können. Die Carbonsäureamide lassen sich dabei unter sehr milden Bedingungen einfach und selektiv "in situ" mit einem Alkylierungsmittel O-alkylieren, somit kann man letztendlich Säureamide selektiv zu Aminen hydrieren. Dabei werden unterschiedlichste funktionelle Gruppen toleriert, unter anderem bleiben Nitrile, Carboxyl- und Phosphon-Gruppen erhalten.

Die technische Aufgabe wird gelöst durch ein Verfahren zur Herstellung von Aminen umfassend die folgenden Schritte:
a) Umsetzung eines
   i. Carbonsäureamids der allgemeinen Formel (I) 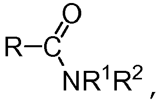 wobei
      R ausgewählt wird aus der Gruppe bestehend aus H, substituiertes oder unsubstituiertes (C₁-C₂₄)-Alkyl, substituiertes oder unsubstituiertes (C₃-C₂₀)-Cycloalkyl, substituiertes oder unsubstituiertes (C₂-C₁₃)-Heterocycloalkyl, substituiertes oder unsubstituiertes (C₆-C₁₄)-Aryl, substituiertes oder unsubstituiertes (C₃-C₁₃)-Heteroaryl und dem Säurerest einer Aminosäure ausgewählt aus Alanin, Arginin, Asparagin, Asparaginsäure, Cystein, Glutamin, Glutaminsäure, Glycin, Histidin, Isoleucin, Leucin, Lysin, Methionin, Phenylalanin, Prolin, Serin, Threonin, Tryptophan, Tyrosin, Valin; und
      R¹ und R² unabhängig voneinander ausgewählt werden aus der Gruppe bestehend aus H, substituiertes oder unsubstituiertes (C₁-C₂₄)-Alkyl, substituiertes oder unsubstituiertes (C₃-C₈)-Cycloalkyl, substituiertes oder unsubstituiertes (C₂-C₇)-Heterocycloalkyl, substituiertes oder unsubstituiertes (C₆-C₁₄)-Aryl oder substituiertes oder unsubstituiertes (C₃-C₁₃)-Heteroaryl, wobei sowohl die Reste R mit R¹, R mit R² als auch R¹ mit R² unabhängig voneinander eine gesättigte oder einfach oder mehrfach ungesättigte (C₂-C₁₈)-Alkylen- oder (C₂-C₁₈)-Heteroalkylen-Brücke bilden, so dass ein aliphatischer oder aromatischer Ring mit insgesamt 3-20 Ringatomen entsteht,
         oder
   ii. Carbonsäurediamids der allgemeinen Formel (II) 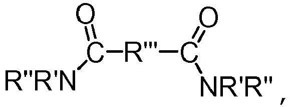 wobei
      R"' ausgewählt wird aus der Gruppe bestehend aus divalenten substituierten oder unsubstituierten (C₁-C₂₄)-Alkylresten, substituierten oder unsubstituierten (C₃-C₂₀)-Cycloalkylresten, substituierten oder unsubstituierten (C₂-C₁₃)-Heterocycloalkylresten, substituierten oder unsubstituierten (C₆-C₁₄)-Arylresten, substituierten oder unsubstituierten (C₃-C₁₃)-Heteroarylresten; und
      R' und R" unabhängig voneinander ausgewählt werden aus der Gruppe bestehend aus H, substituiertes oder unsubstituiertes (C₁-C₂₄)-Alkyl, substituiertes oder unsubstituiertes (C₁-C₂₄)-Heteroalkyl, substituiertes oder unsubstituiertes (C₃-C₈)-Cycloalkyl, substituiertes oder unsubstituiertes (C₂-C₇)-Heterocycloalkyl, substituiertes oder unsubstituiertes (C₆-C₁₄)-Aryl oder substituiertes oder unsubstituiertes (C₃-C₁₃)-Heteroaryl, wobei die Reste R' mit R" miteinander eine gesättigte oder einfach oder mehrfach ungesättigte (C₂-C₁₈)-Alkylen- oder (C₂-C₁₈)-Heteroalkylen-Brücke bilden können, so dass ein aliphatischer oder aromatischer Ring mit insgesamt 3-20 Ringatomen entsteht,
         oder
   iii. Di-, Tri- oder Polypeptids, oder
   iv. Peptidamids mit carboxyterminaler Amidfunktion
   mit einem Alkylierungsmittel,
b) Zugabe eines Hydrierkatalysators zum Reaktionsgemisch, wobei das Mol-Verhältnis von Hydrierkatalysator, der mindestens ein aktives Metall der Gruppe VII B und/oder VIII B des Periodensystems der Elemente enthält, zu Carbonsäureamid oder Carbonsäurediamid oder Di-, Tri- oder Polypeptid oder Peptidamid in einem Bereich von 1:10 bis 1:100.000 liegt,
c) Umsetzung des Reaktionsgemisches mit Wasserstoff, wobei ein Wasserstoffdruck von 0,1 bar bis 200 bar eingestellt wird und wobei eine Temperatur in einem Bereich von 0°C bis 120°C eingestellt wird.

Im Sinne der vorliegenden Erfindung bezeichnet der Begriff Amine alle Verbindungen, die die folgende Struktureinheit 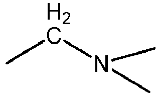 aufweisen, wobei es unerheblich ist, welche weiteren funktionellen Gruppen die Verbindung aufweist. Insbesondere sind unter Aminen die Reaktionsprodukte zu verstehen, die durch die Hydrierung von Amidfunktionen entstehen:

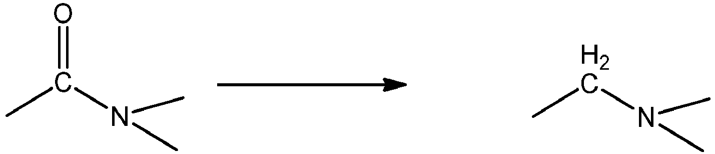

Im Sinne der vorliegenden Erfindung bezeichnet der Begriff Carbonsäureamid Verbindungen der allgemeinen Formel (I) 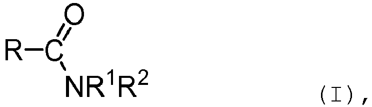 wobei
R ausgewählt wird aus der Gruppe bestehend aus H, substituiertes oder unsubstituiertes (C₁-C₂₄)-Alkyl, substituiertes oder unsubstituiertes (C₃-C₂₀)-Cycloalkyl, substituiertes oder unsubstituiertes (C₂-C₁₃)-Heterocycloalkyl, substituiertes oder unsubstituiertes (C₆-C₁₄)-Aryl, substituiertes oder unsubstituiertes (C₃-C₁₃)-Heteroaryl und dem Säurerest einer Aminosäure ausgewählt aus Alanin, Arginin, Asparagin, Asparaginsäure, Cystein, Glutamin, Glutaminsäure, Glycin, Histidin, Isoleucin, Leucin, Lysin, Methionin, Phenylalanin, Prolin, Serin, Threonin, Tryptophan, Tyrosin, Valin; und
R¹ und R² unabhängig voneinander ausgewählt werden aus der Gruppe bestehend aus H, substituiertes oder unsubstituiertes (C₁-C₂₄)-Alkyl, substituiertes oder unsubstituiertes (C₃-C₈)-Cycloalkyl, substituiertes oder unsubstituiertes (C₂-C₇)-Heterocycloalkyl, substituiertes oder unsubstituiertes (C₆-C₁₄)-Aryl oder substituiertes oder unsubstituiertes (C₃-C₁₃)-Heteroaryl, wobei sowohl die Reste R mit R¹, R mit R² als auch R¹ mit R² unabhängig voneinander eine gesättigte oder einfach oder mehrfach ungesättigte (C₂-C₁₈)-Alkylen- oder (C₂-C₁₈)-Heteroalkylen-Brücke bilden, so dass ein aliphatischer oder aromatischer Ring mit insgesamt 3-20 Ringatomen entsteht.

R ist bevorzugt ausgewählt aus der Gruppe bestehend aus H, (C₁-C₂₄)-Alkyl, Phenyl, und dem Säurerest einer Aminosäure ausgewählt aus Alanin, Arginin, Asparagin, Asparaginsäure, Cystein, Glutamin, Glutaminsäure, Glycin, Histidin, Isoleucin, Leucin, Lysin, Methionin, Phenylalanin, Prolin, Serin, Threonin, Tryptophan, Tyrosin, Valin; R wird besonders bevorzugt ausgewählt aus H, Methyl, Ethyl, n-Propyl, n-Butyl, iso-Butyl, n-Pentyl, n-Hexyl, Cyclohexyl, n-Heptyl, n-Octyl, Phenyl, Pyridyl, Naphthyl, -C₂H₄PO(OEt)₂, -C₈H₁₆-CH=CH₂, -COOEt,
N-Boc-aminomethyl, N-Boc-1-aminoethyl, N-Boc-1-amino-2-methylpropyl, N-Boc-1-amino-3-methylbutyl, N-Boc-1-amino-3-methylthiopropyl, N-Boc-1-amino-2-phenylethyl und N-Bocpyrrolidinyl.

R¹ und R² sind bevorzugt ausgewählt aus der Gruppe bestehend aus Wasserstoff, Methyl, Ethyl, n-Propyl, iso-Propyl, n-Butyl, iso-Butyl, n-Pentyl, n-Hexyl, n-Heptyl, n-Octyl, und Phenyl.

Ein Ring wird bevorzugt zwischen den Resten R und R¹ oder R¹ und R² gebildet, wobei der Ring bevorzugt aliphatisch ist und die Alkylengruppen Propandi-1,3-yl, Butandi-1,4-yl, Pentandi-1,5-yl, oder Hexandi-1,6-yl aufweist, so dass der Ring insgesamt 4, 5, 6, 7 oder 8 Ringatome enthält.

Bevorzugt gebildete Ringe sind Pyrrolidin, Piperidin, Morpholin, Piperazin, Homopiperidin und Homopiperazin sowie deren Derivate.

Bevorzugte Carbonsäureamide können Tabelle 1 entnommen werden.

**Tabelle 1 : Bevorzugte Carbonsäureamide.**

| **Verbindung** | **R** | **R¹** | **R²** |
|---|---|---|---|
| 1 | H | Methyl | Methyl |
| 2 | H | Ethyl | Ethyl |
| 3 | H | n-Propyl | n-Propyl |
| 4 | H | iso-Propyl | iso-Propyl |
| 5 | H | n-Butyl | n-Butyl |
| 6 | H | iso-Butyl | iso-Butyl |
| 7 | H | n-Pentyl | n-Pentyl |
| 8 | H | n-Hexyl | n-Hexyl |
| 9 | H | n-Heptyl | n-Heptyl |
| 10 | H | n-Octyl | n-Octyl |
| 11 | H | Phenyl | Phenyl |
| 12 | Methyl | Methyl | Methyl |
| 13 | Methyl | Ethyl | Ethyl |
| 14 | Methyl | n-Propyl | n-Propyl |
| 15 | Methyl | iso-Propyl | iso-Propyl |
| 16 | Methyl | n-Butyl | n-Butyl |
| 17 | Methyl | iso-Butyl | iso-Butyl |
| 18 | Methyl | n-Pentyl | n-Pentyl |
| 19 | Methyl | n-Hexyl | n-Hexyl |
| 20 | Methyl | n-Heptyl | n-Heptyl |
| 21 | Methyl | n-Octyl | n-Octyl |
| 22 | Methyl | Phenyl | Phenyl |
| 23 | Ethyl | Methyl | Methyl |
| 24 | Ethyl | Ethyl | Ethyl |
| 25 | Ethyl | n-Propyl | n-Propyl |
| 26 | Ethyl | iso-Propyl | iso-Propyl |
| 27 | Ethyl | n-Butyl | n-Butyl |
| 28 | Ethyl | iso-Butyl | iso-Butyl |
| 29 | Ethyl | n-Pentyl | n-Pentyl |
| 30 | Ethyl | n-Hexyl | n-Hexyl |
| 31 | Ethyl | n-Heptyl | n-Heptyl |
| 32 | Ethyl | n-Octyl | n-Octyl |
| 33 | Ethyl | Phenyl | Phenyl |
| 34 | n-Propyl | Methyl | Methyl |
| 35 | n-Propyl | Ethyl | Ethyl |
| 36 | n-Propyl | n-Propyl | n-Propyl |
| 37 | n-Propyl | iso-Propyl | iso-Propyl |
| 38 | n-Propyl | n-Butyl | n-Butyl |
| 39 | n-Propyl | iso-Butyl | iso-Butyl |
| 40 | n-Propyl | n-Pentyl | n-Pentyl |
| 41 | n-Propyl | n-Hexyl | n-Hexyl |
| 42 | n-Propyl | n-Heptyl | n-Heptyl |
| 43 | n-Propyl | n-Octyl | n-Octyl |
| 44 | n-Propyl | Phenyl | Phenyl |
| 45 | n-Butyl | Methyl | Methyl |
| 46 | n-Butyl | Ethyl | Ethyl |
| 47 | n-Butyl | n-Propyl | n-Propyl |
| 48 | n-Butyl | iso-Propyl | iso-Propyl |
| 49 | n-Butyl | n-Butyl | n-Butyl |
| 50 | n-Butyl | iso-Butyl | iso-Butyl |
| 51 | n-Butyl | n-Pentyl | n-Pentyl |
| 52 | n-Butyl | n-Hexyl | n-Hexyl |
| 53 | n-Butyl | n-Heptyl | n-Heptyl |
| 54 | n-Butyl | n-Octyl | n-Octyl |
| 55 | n-Butyl | Phenyl | Phenyl |
| 56 | iso-Butyl | Methyl | Methyl |
| 57 | iso-Butyl | Ethyl | Ethyl |
| 58 | iso-Butyl | n-Propyl | n-Propyl |
| 59 | iso-Butyl | iso-Propyl | iso-Propyl |
| 60 | iso-Butyl | n-Butyl | n-Butyl |
| 61 | iso-Butyl | iso-Butyl | iso-Butyl |
| 62 | iso-Butyl | n-Pentyl | n-Pentyl |
| 63 | iso-Butyl | n-Hexyl | n-Hexyl |
| 64 | iso-Butyl | n-Heptyl | n-Heptyl |
| 65 | iso-Butyl | n-Octyl | n-Octyl |
| 66 | iso-Butyl | Phenyl | Phenyl |
| 67 | n-Pentyl | Methyl | Methyl |
| 68 | n-Pentyl | Ethyl | Ethyl |
| 69 | n-Pentyl | n-Propyl | n-Propyl |
| 70 | n-Pentyl | iso-Propyl | iso-Propyl |
| 71 | n-Pentyl | n-Butyl | n-Butyl |
| 72 | n-Pentyl | iso-Butyl | iso-Butyl |
| 73 | n-Pentyl | n-Pentyl | n-Pentyl |
| 74 | n-Pentyl | n-Hexyl | n-Hexyl |
| 75 | n-Pentyl | n-Heptyl | n-Heptyl |
| 76 | n-Pentyl | n-Octyl | n-Octyl |
| 77 | n-Pentyl | Phenyl | Phenyl |
| 78 | n-Hexyl | Methyl | Methyl |
| 79 | n-Hexyl | Ethyl | Ethyl |
| 80 | n-Hexyl | n-Propyl | n-Propyl |
| 81 | n-Hexyl | iso-Propyl | iso-Propyl |
| 82 | n-Hexyl | n-Butyl | n-Butyl |
| 83 | n-Hexyl | iso-Butyl | iso-Butyl |
| 84 | n-Hexyl | n-Pentyl | n-Pentyl |
| 85 | n-Hexyl | n-Hexyl | n-Hexyl |
| 86 | n-Hexyl | n-Heptyl | n-Heptyl |
| 87 | n-Hexyl | n-Octyl | n-Octyl |
| 88 | n-Hexyl | Phenyl | Phenyl |
| 89 | Cyclohexyl | Methyl | Methyl |
| 90 | Cyclohexyl | Ethyl | Ethyl |
| 91 | Cyclohexyl | n-Propyl | n-Propyl |
| 92 | Cyclohexyl | iso-Propyl | iso-Propyl |
| 93 | Cyclohexyl | n-Butyl | n-Butyl |
| 94 | Cyclohexyl | iso-Butyl | iso-Butyl |
| 95 | Cyclohexyl | n-Pentyl | n-Pentyl |
| 96 | Cyclohexyl | n-Hexyl | n-Hexyl |
| 97 | Cyclohexyl | n-Heptyl | n-Heptyl |
| 98 | Cyclohexyl | n-Octyl | n-Octyl |
| 99 | Cyclohexyl | Phenyl | Phenyl |
| 100 | n-Heptyl | Methyl | Methyl |
| 101 | n-Heptyl | Ethyl | Ethyl |
| 102 | n-Heptyl | n-Propyl | n-Propyl |
| 103 | n-Heptyl | iso-Propyl | iso-Propyl |
| 104 | n-Heptyl | n-Butyl | n-Butyl |
| 105 | n-Heptyl | iso-Butyl | iso-Butyl |
| 106 | n-Heptyl | n-Pentyl | n-Pentyl |
| 107 | n-Heptyl | n-Hexyl | n-Hexyl |
| 108 | n-Heptyl | n-Heptyl | n-Heptyl |
| 109 | n-Heptyl | n-Octyl | n-Octyl |
| 110 | n-Heptyl | Phenyl | Phenyl |
| 111 | n-Octyl | Methyl | Methyl |
| 112 | n-Octyl | Ethyl | Ethyl |
| 113 | n-Octyl | n-Propyl | n-Propyl |
| 114 | n-Octyl | iso-Propyl | iso-Propyl |
| 115 | n-Octyl | n-Butyl | n-Butyl |
| 116 | n-Octyl | iso-Butyl | iso-Butyl |
| 117 | n-Octyl | n-Pentyl | n-Pentyl |
| 118 | n-Octyl | n-Hexyl | n-Hexyl |
| 119 | n-Octyl | n-Heptyl | n-Heptyl |
| 120 | n-Octyl | n-Octyl | n-Octyl |
| 121 | n-Octyl | Phenyl | Phenyl |
| 122 | Phenyl | Methyl | Methyl |
| 123 | Phenyl | Ethyl | Ethyl |
| 124 | Phenyl | n-Propyl | n-Propyl |
| 125 | Phenyl | iso-Propyl | iso-Propyl |
| 126 | Phenyl | n-Butyl | n-Butyl |
| 127 | Phenyl | iso-Butyl | iso-Butyl |
| 128 | Phenyl | n-Pentyl | n-Pentyl |
| 129 | Phenyl | n-Hexyl | n-Hexyl |
| 130 | Phenyl | n-Heptyl | n-Heptyl |
| 131 | Phenyl | n-Octyl | n-Octyl |
| 132 | Phenyl | Phenyl | Phenyl |
| 133 | Pyridyl | Methyl | Methyl |
| 134 | Pyridyl | Ethyl | Ethyl |
| 135 | Pyridyl | n-Propyl | n-Propyl |
| 136 | Pyridyl | iso-Propyl | iso-Propyl |
| 137 | Pyridyl | n-Butyl | n-Butyl |
| 138 | Pyridyl | iso-Butyl | iso-Butyl |
| 139 | Pyridyl | n-Pentyl | n-Pentyl |
| 140 | Pyridyl | n-Hexyl | n-Hexyl |
| 141 | Pyridyl | n-Heptyl | n-Heptyl |
| 142 | Pyridyl | n-Octyl | n-Octyl |
| 143 | Pyridyl | Phenyl | Phenyl |
| 144 | Naphthyl | Methyl | Methyl |
| 145 | Naphthyl | Ethyl | Ethyl |
| 146 | Naphthyl | n-Propyl | n-Propyl |
| 147 | Naphthyl | iso-Propyl | iso-Propyl |
| 148 | Naphthyl | n-Butyl | n-Butyl |
| 149 | Naphthyl | iso-Butyl | iso-Butyl |
| 150 | Naphthyl | n-Pentyl | n-Pentyl |
| 151 | Naphthyl | n-Hexyl | n-Hexyl |
| 152 | Naphthyl | n-Heptyl | n-Heptyl |
| 153 | Naphthyl | n-Octyl | n-Octyl |
| 154 | Naphthyl | Phenyl | Phenyl |
| 155 | -C₂H₄PO(OEt) ₂ | Methyl | Methyl |
| 156 | -C₈H₁₆-CH=CH₂(1-Decenyl?) | Methyl | Methyl |
| 157 | -C(=O)OEt | Methyl | Methyl |
| 158 | - (CH₂)₂⁻ C(=O)NMe₂ | Methyl | Methyl |
| 159 | H | (CH₂)₃⁻ | |
| 160 | Methyl | -(CH₂)₃- | |
| 161 | Ethyl | - (CH₂)₃- | |
| 162 | Propyl | -(CH₂)₃- | |
| 163 | Butyl | - (CH₂)₃- | |
| 164 | n-Pentyl | - (CH₂)₃- | |
| 165 | n-Hexyl | - (CH₂)₃- | |
| 166 | Cyclohexyl | -(CH₂)₃- | |
| 167 | n-Heptyl | - (CH₂) ₃- | |
| 168 | n-Octyl | -(CH₂)₃- | |
| 169 | Pyridyl | -(CH₂)₃- | |
| 170 | Naphthyl | -(CH₂) ₃- | |
| 171 | Phenyl | -(CH₂)₃- | |
| 172 | H | - (CH₂) ₄- | |
| 173 | Methyl | -(CH₂)₄- | |
| 174 | Ethyl | - (CH₂) ₄- | |
| 175 | Propyl | - (CH₂) ₄- | |
| 176 | Butyl | - (CH₂)₄- | |
| 177 | n-Pentyl | - (CH₂)₄- | |
| 178 | n-Hexyl | - (CH₂)₄- | |
| 179 | Cyclohexyl | -(CH₂)₄- | |
| 180 | n-Heptyl | - (CH₂)₄- | |
| 181 | n-Octyl | - (CH₂)₄- | |
| 182 | Pyridyl | - (CH₂)₄- | |
| 183 | Naphthyl | - (CH₂)₄- | |
| 184 | Phenyl | - (CH₂)₄- | |
| 185 | H | - (CH₂)₅- | |
| 186 | Methyl | -(CH₂)₅- | |
| 187 | Ethyl | -(CH₂)₅⁻ | |
| 188 | Propyl | -(CH₂)₅- | |
| 189 | Butyl | -(CH₂)₅- | |
| 190 | n-Pentyl | -(CH₂)₅- | |
| 191 | n-Hexyl | -(CH₂)₅- | |
| 192 | Cyclohexyl | -(CH₂)₅- | |
| 193 | n-Heptyl | - (CH₂)₅- | |
| 194 | n-Octyl | -(CH₂)₅- | |
| 195 | Pyridyl | -(CH₂)₅- | |
| 196 | Naphthyl | -(CH₂)₅- | |
| 197 | Phenyl | -(CH₂)₅- | |
| 198 | H | -(CH₂)₆- | |
| 199 | Methyl | -(CH₂)₆- | |
| 200 | Ethyl | -(CH₂)₆- | |
| 201 | Butyl | -(CH₂)₆- | |
| 202 | Butyl | -(CH₂)₆- | |
| 203 | n-Pentyl | -(CH₂)₆- | |
| 204 | n-Hexyl | -(CH₂)₆- | |
| 205 | Cyclohexyl | -(CH₂)₆- | |
| 206 | n-Heptyl | -(CH₂)₆- | |
| 207 | n-Octyl | -(CH₂)₆- | |
| 208 | Pyridyl | -(CH₂)₆- | |
| 209 | Naphthyl | -(CH₂)₆- | |
| 210 | Phenyl | -(CH₂)₆- | |
| 211 | -(CH₂)₃- | | -(CH₂)₂-CN |
| 212 | -(CH₂)₃- | | H |
| 213 | -(CH₂)₃- | | Methyl |
| 214 | -(CH₂)₄- | | H |
| 215 | -(CH₂)₄- | | Methyl |
| 216 | -(CH₂)₅- | | H |
| 217 | -(CH₂)₅- | | Methyl |
| 218 | -C (=O)O-(CH₂)₂- | | H |
| 219 | -C (=O)O-(CH₂)₂- | | Methyl |
| 220 | - (CH₂) ₂-NH-(CH₂)₂- | | H |
| 221 | - (CH₂) ₂-NH-(CH₂)₂- | | Methyl |
| 222 | - (CH₂) ₃-NH-(CH₂)₂- | | H |
| 223 | - (CH₂) ₃-NH-(CH₂)₂- | | Methyl |

Im Sinne der vorliegenden Erfindung bezeichnet der Begriff Carbonsäurediamid Verbindungen der allgemeinen Formel (II) 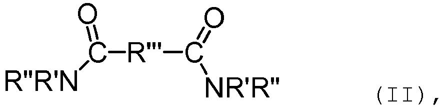 wobei
R'" ausgewählt wird aus der Gruppe bestehend aus divalenten substituierten oder unsubstituierten (C₁-C₂₄)-Alkylresten, substituierten oder unsubstituierten (C₃-C₂₀)-Cycloalkylresten, substituierten oder unsubstituierten (C₂-C₁₃)-Heterocycloalkylresten, substituierten oder unsubstituierten (C₆-C₁₄)-Arylresten, substituierten oder unsubstituierten (C₃-C₁₃)-Heteroarylresten; und R' und R" unabhängig voneinander ausgewählt werden aus der Gruppe bestehend aus H, substituiertes oder unsubstituiertes (C₁-C₂₄)-Alkyl, substituiertes oder unsubstituiertes (C₁-C₂₄)-Heteroalkyl, substituiertes oder unsubstituiertes (C₃-C₈)-Cycloalkyl, substituiertes oder unsubstituiertes (C₂-C₇)-Heterocycloalkyl, substituiertes oder unsubstituiertes (C₆-C₁₄)-Aryl oder substituiertes oder unsubstituiertes (C₃-C₁₃)-Heteroaryl, wobei die Reste R' mit R" miteinander eine gesättigte oder einfach oder mehrfach ungesättigte (C₂-C₁₈)-Alkylen- oder (C₂-C₁₈)-Heteroalkylen-Brücke bilden können, so dass ein aliphatischer oder aromatischer Ring mit insgesamt 3-20 Ringatomen entsteht.
R"' ist bevorzugt ausgewählt aus divalenten (C₁-C₂₄)-Alkylresten, wobei die Gruppe bestehend aus Methylen, Ethylen, n-Propylen, n-Butylen, n-Pentylen, und n-Hexylen bevorzugt ist.
R' und R" sind bevorzugt unabhängig voneinander ausgewählt aus der Gruppe bestehend aus Wasserstoff, Methyl, Ethyl, n-Propyl, iso-Propyl, n-Butyl, iso-Butyl, n-Pentyl, n-Hexyl, n-Heptyl, n-Octyl, Butandi-1,4-yl, Pentandi-1,5-yl, Hexandi-1,6-yl und Phenyl.
   Ein Ring zwischen den Resten R' und R" ist bevorzugt aliphatisch und weist insgesamt 5, 6 oder 7 Ringatome aufweist. Bevorzugt gebildete Ringe sind Pyrrolidin, Piperidin, Morpholin, Piperazin, Homopiperidin und Homopiperazin sowie deren Derivate.

Bevorzugte Carbonsäurediamide sind N, N, N', N'-Tetramethyl-bernsteinsäurediamid, N, N, N', N'-Tetramethyl-adipinsäurediamid, N, N, N',N'-Tetramethyl-korksäurediamid, N, N, N', N'-Tetramethyl-terephthalsäurediamid und Sarcosinanhydrid.

Im Sinne der vorliegenden Erfindung bezeichnet der Begriff Di-, Tri- oder Polypeptid Verbindungen, die eine Peptidbindung zwischen Aminosäuren enthalten. Ein Dipeptid bezeichnet dabei die Verbindung zwischen zwei Aminosäuren AS¹-AS², ein Tripeptid bezeichnet die Verbindung zwischen drei Aminosäuren AS¹-AS²-AS³ und ein Polypeptid bezeichnet die Verbindung zwischen vier bis über 100 Aminosäuren. Die Aminosäuren AS¹, AS² und AS³ können jeweils unabhängig voneinander aus der Gruppe bestehend aus Alanin, Arginin, Asparagin, Asparaginsäure, Cystein, Glutamin, Glutaminsäure, Glycin, Histidin, Isoleucin, Leucin, Lysin, Methionin, Phenylalanin, Prolin, Serin, Threonin, Tryptophan, Tyrosin, Valin ausgewählt werden, wobei die D- und L-Enantiomere umfasst sind. Bei der Kondensation von Aminosäuren reagiert die Carboxylgruppe der einen Aminosäure formal unter Wasserabspaltung mit der Aminogruppe der anderen Aminosäure zur Säureamidgruppierung -C(=O)-NH, die neu geknüpfte Amidbindung zwischen dem Kohlenstoff der Carbonylgruppe und dem Stickstoffatom wird Peptidbindung genannt.
Die freie Aminogruppe an einem Ende des Peptids nennt man N-Terminus, die freie Carboxylgruppe am anderen Ende wird C-Terminus oder Carboxy-Terminus genannt. Der Carboxy-Terminus ist bevorzugt alkyliert oder silyliert, wobei bevorzugt mit Methyl oder Ethyl, MeOCH₂-, Tetrahydropyranyl(THP), PhCOCH₂-, *t*-Bu, Allyl, Benzyl(Bn), Ph₂CH- alkyliert ist oder mit Me₃Si- oder *t*-BuMe₂Si-(TBDMS) silyliert ist; der N-Terminus ist bevorzugt mit einer Schutzgruppe versehen, wobei die Schutzgruppe bevorzugt aus der folgenden Gruppe bestehend aus Boc, Cbz, Fmoc und Alloc ausgewählt wird, wobei Boc=tert-Butyloxycarbonyl, Cbz=Benzyloxycarbonyl, Fmoc=Fluorenylmethylenoxycarbonyl, Alloc=Allyloxycarbonyl bedeutet.

Beispiele für Dipeptide können Tabelle 2 entnommen werden.

**Tabelle 2: Bevorzugte Dipeptide AS¹-AS², wobei die Aminosäure AS¹ in der ersten Spalte im 3-Buchstabencode angegeben ist und die Aminosäure AS² in der ersten Zeile im 3-Buchstabencode angegeben ist, und wobei jeweils die D- und L-Enantiomere umfasst sind.**

| | **Ala** | **Arg** | **Asn** | **Asp** | **Cys** | **Gln** | **Glu** | **His** | **Ile** | **Leu** | **Lys** | **Met** | **Phe** | **Pro** | **Ser** | **Thr** | **Trp** | **Tyr** | **Val** |
|---|---|---|---|---|---|---|---|---|---|---|---|---|---|---|---|---|---|---|---|
| **Ala** | x | x | x | x | x | x | x | x | x | x | x | x | x | x | x | x | x | x | x |
| **Arg** | x | x | x | x | x | x | x | x | x | x | x | x | x | x | x | x | x | x | x |
| **Asn** | x | x | x | x | x | x | x | x | x | x | x | x | x | x | x | x | x | x | x |
| **Asp** | x | x | x | x | x | x | x | x | x | x | x | x | x | x | x | x | x | x | x |
| **Cys** | x | x | x | x | x | x | x | x | x | x | x | x | x | x | x | x | x | x | x |
| **Gln** | x | x | x | x | x | x | x | x | x | x | x | x | x | x | x | x | x | x | x |
| **Glu** | x | x | x | x | x | x | x | x | x | x | x | x | x | x | x | x | x | x | x |
| **Gly** | x | x | x | x | x | x | x | x | x | x | x | x | x | x | x | x | x | x | x |
| **His** | x | x | x | x | x | x | x | x | x | x | x | x | x | x | x | x | x | x | x |
| **Ile** | x | x | x | x | x | x | x | x | x | x | x | x | x | x | x | x | x | x | x |
| **Leu** | x | x | x | x | x | x | x | x | x | x | x | x | x | x | x | x | x | x | x |
| **Lys** | x | x | x | x | x | x | x | x | x | x | x | x | x | x | x | x | x | x | x |
| **Met** | x | x | x | x | x | x | x | x | x | x | x | x | x | x | x | x | x | x | x |
| **Phe** | x | x | x | x | x | x | x | x | x | x | x | x | x | x | x | x | x | x | x |
| **Pro** | x | x | x | x | x | x | x | x | x | x | x | x | x | x | x | x | x | x | x |
| **Ser** | x | x | x | x | x | x | x | x | x | x | x | x | x | x | x | x | x | x | x |
| **Thr** | x | x | x | x | x | x | x | x | x | x | x | x | x | x | x | x | x | x | x |
| **Trp** | x | x | x | x | x | x | x | x | x | x | x | x | x | x | x | x | x | x | x |
| **Tyr** | x | x | x | x | x | x | x | x | x | x | x | x | x | x | x | x | x | x | x |
| **Val** | x | x | x | x | x | x | x | x | x | x | x | x | x | x | x | x | x | x | x |

Im Sinne der vorliegenden Erfindung bezeichnet der Begriff Peptidamid mit carboxyterminaler Amidfunktion ein Di-, Tri- oder Polypeptid, das am Carboxy-Terminus eine Amidfunktion -NR¹R² aufweist, wobei R¹ und R² unabhängig voneinander ausgewählt werden aus H, substituiertes oder unsubstituiertes (C₁-C₂₄)-Alkyl, substituiertes oder unsubstituiertes (C₃-C₈)-Cycloalkyl, substituiertes oder unsubstituiertes (C₂-C₇)-Heterocycloalkyl, substituiertes oder unsubstituiertes (C₆-C₁₄)-Aryl oder substituiertes oder unsubstituiertes (C₃-C₁₃)-Heteroaryl.

Unter einem (C₁-Cₙ)-Alkylrest sind sowohl lineare als auch verzweigte Alkylreste mit 1 bis n C-Atomen zu verstehen. Bei verzweigten Alkylresten kann die Verzweigung an einem beliebigen Kohlenstoff-Atom auftreten.
Bevorzugte (C₁-Cₙ)-Alkylreste sind Methyl, Ethyl, n-Propyl, iso-Propyl, n-Butyl, iso-Butyl, sec-Butyl, n-Pentyl, isoPentyl, n-Hexyl, n-Heptyl, n-Octyl, n-Nonyl, n-Decyl, n-Dodecyl und n-Octadecyl.
Der (C₁-Cₙ)-Alkylrest kann substituiert oder unsubstituiert sein, bevorzugt ist er unsubstituiert.

Unter einem (C₁-Cₙ)-Heteroalkylrest sind sowohl lineare als auch verzweigte Alkylreste mit 1 bis n C-Atomen zu verstehen, wobei 1 oder 2 C-Atome durch Heteroatome ausgewählt aus der Gruppe N, O, P ersetzt sind. Bei verzweigten Heteroalkylresten kann die Verzweigung an einem beliebigen Kohlenstoff-Atom auftreten.
Der (C₁-Cₙ)-Heteroalkylrest kann substituiert oder unsubstituiert sein, bevorzugt ist er unsubstituiert.

Ein (C₃-Cₙ)-Cycloalkylrest bezeichnet ein mono-, bi- oder tricyclisches, aliphatisches System aus insgesamt 3 bis n C-Atomen, wobei jeder Ring drei-, vier-, fünf-, sechs- oder siebengliedrig sein kann. Bevorzugt sind (C₆-C₁₂)-Cycloalkylreste. Besonders bevorzugte (C₃-Cₙ)-Cycloalkylreste sind Cyclopropyl, Cyclobutyl, Cyclopentyl, Cyclohexyl, Cycloheptyl und 1-Adamantyl, 9-Fluorenyl.
Der (C₃-Cₙ)-Cycloalkylrest kann substituiert oder unsubstituiert sein, bevorzugt ist er unsubstituiert.

Ein (C₂-Cₙ)-Heterocycloalkylrest bezeichnet ein mono-, bi- oder tricyclisches, aliphatisches System aus insgesamt 2 bis n C-Atomen, wobei jeder Ring drei-, vier-, fünf-, sechs- oder siebengliedrig sein kann, und wobei die Anzahl der Heteroatome, ausgewählt aus der Gruppe N, O, und S 1 oder 2 beträgt und die Heteroatome gleich oder verschieden sind. Bevorzugte Heterocycloalkylreste sind 2-, 3-Tetrahydrofuryl, 1-, 2-, 3-Pyrrolidinyl, 1-, 2-, 3-, 4-Piperidinyl, 1-, 2-Piperazinyl, 1-, 2-, 3-Morpholinyl, Tetrahydropyranyl-2 oder - 3 und 2,3-Dihydro-benzothiophenyl-2 oder -3.
Der (C₂-Cₙ)-Heterocycloalkylrest kann substituiert oder unsubstituiert sein, bevorzugt ist er unsubstituiert.

Ein (C₆-Cₙ)-Arylrest bezeichnet ein mono-, bi- und tricyclisches, aromatisches System mit 6 bis n C-Atomen, wobei jeder Ring jeweils fünf-, sechs- oder siebengliedrig sein kann. Bevorzugte (C₆-Cₙ)-Arylreste sind Phenyl, Naphthyl, Anthryl, Phenantryl, Biphenyl.
Der (C₆-Cₙ)-Arylrest kann substituiert oder unsubstituiert sein, bevorzugt ist er unsubstituiert.

Ein (C₃-Cₙ)-Heteroarylrest bezeichnet ein mono-, bi- oder tricyclisches, aromatisches System aus insgesamt 3 bis n C-Atomen, wobei jeder Ring jeweils fünf-, sechs- oder siebengliedrig sein kann, und wobei die Anzahl der Heteroatome, ausgewählt aus der Gruppe N, O, und S 1 oder 2 beträgt und die Heteroatome gleich oder verschieden sind. Bevorzugte (C₂-Cₙ)-Heteroarylreste sind 2-, 3-Furyl, 2-, 3-Pyrrolyl, 2-, 4-, 5-Imidazolyl, 2-, 3-Thienyl, 2-, 3-, 4-Pyridyl, 2-, 3-, 4-, 5-, 6-, 7-Indolyl, 3-, 4-, 5-Pyrazolyl, 2-, 4-, 5-, 6-Pyrimidinyl, Acridinyl, Chinolinyl, Phenantridinyl, Benzothienyl.
Der (C₃-Cₙ)-Heteroarylrest kann substituiert oder unsubstituiert sein, bevorzugt ist er unsubstituiert.

Substituenten werden ausgewählt aus der Gruppe bestehend aus Halogenen wie F, Cl, Br, I, und heteroatomhaltigen funktionellen Gruppen, die ein oder mehrere Atome ausgewählt aus der Gruppe bestehend aus N, O, P, S, oder Si enthalten, wobei Einfach- und Mehrfachsubstitution möglich ist. Beispiele für heteroatomhaltige funktionelle Gruppen sind Carbonyl-, Carboxyl-, Sulphonat-, Phosphonat-, Hydroxyl-, Amino-, Ammoniumgruppen wie

-OH,

- (C₁-C₈)-Alkyloxy

-COOH,

-NH ({C₁-C₈}-Acyl),

-NH ({C₁-C₈}-Acyloxy)

-N((C₁-C₂₀)-Alkyl) ({C₁-C₈}-Acyl),

-N({C₆-C₁₄}-Aryl)({C₁-C₈}-Acyl),

-N({C₆-C₁₄}-Aralkyl)({C₁-C₈}-Acyl),

-N({C₁-C₈}-Acyl)₂,

-NH₃⁺,

-NH({C₁-C₂₀}-Alkyl)₂⁺,

-NH({C₆-C₁₄}-Aryl)₂⁺,

-NH({C₆-C₁₄}-Aralkyl)₂⁺,

-NH({C₁-C₂₀}-Alkyl)({C₆-C₁₄}-Aryl)⁺,

-N({C₆-C₁₄}-Aryl)({C₁-C₂₀}-Alkyl)₂⁺,

-N({C₆-C₁₄}-Aryl)₂({C₁-C₂₀}-Alkyl)⁺,

-O-C(=O)-O-{C₁-C₂₀}-Alkyl,

-O-C(=O)-O-{C₆-C₁₄}-Aryl,

-O-C(=O)-O-{C₆-C₁₄}-Aralkyl,

-NH-C(=O)-O-{C₁-C₂₀}-Alkyl,

-NH-C(=O)-O-{C₆-C₁₄}-Aryl,

-NH-C(=O)-O-{C₆-C₁₄}-Aralkyl,

-O-C(=O)-NH-{C₁-C₂₀}-Alkyl,

-O-C(=O)-NH-{C₆-C₁₄}-Aryl,

-O-C(=O)-NH-{C₆-C₁₄}-Aralkyl,

-CN,

-SO₂-O-{C₁-C₂₀}-Alkyl,

-SO₂-O-{C₆-C₁₄}-Aryl,

-SO₂-O-{C₆-C₁₄}-Aralkyl,

-SO₂-{C₁-C₂₀}-Alkyl,

-SO₂-{C₆-C₁₄}-Aryl,

-SO₂-{C₆-C₁₄}-Aralkyl,

-SO-{C₁-C₂₀}-Alkyl,

-SO-{C₆-C₁₄}-Aryl,

-SO-{C₆-C₁₄}-Aralkyl,

-Si({C₁-C₂₀}-Alkyl)₃,

-Si({C₆-C₁₄}-Aryl)₃,

-Si({C₆-C₁₄}-Aryl)({C₁-C₂₀}-Alkyl)₂,

-Si({C₆-C₁₄}-Aryl)₂({C₁-C₂₀}-Alkyl),

-{C₁-C₂₀}-Perfluoroalkyl,

-PO(O-{C₁-C₂₀}-Alkyl)₂,

-PO(O-{C₆-C₁₄}-Aryl)₂,

-PO(O-{C₁-C₂₀}-Alkyl)(O-{C₆-C₁₄}-Aryl),

-PO({C₁-C₂₀}-Alkyl)₂,

-PO({C₆-C₁₄}-Aryl)₂,

-PO({C₁-C₂₀}-Alkyl)({C₆-C₁₄}-Aryl).

Im Sinne der vorliegenden Erfindung ist (C₁-Cₙ)-Alkyloxy definiert als lineare oder verzweigte (C₁-Cₙ)-Alkylgruppe mit 1 bis n C-Atomen mit der Maßgabe, dass diese über ein Sauerstoffatom an das diese Gruppe tragende Molekül gebunden ist.

Im Sinne der vorliegenden Erfindung ist (C₁-Cₙ)-Acyl definiert als eine Gruppe mit der allgemeinen Struktur R-(C=O)- mit insgesamt 1 bis n Kohlenstoffatomen, wobei R ausgewählt wird aus der Gruppe bestehend aus H, (C₁-Cₙ₋₁)-Alkyl, (C₁-Cₙ₋₁)-Alkenyl, (C₆-Cₙ₋₁)-Aryl, (C₆-Cₙ₋₁)-Heteroaryl und (C₂-Cₙ₋₁)-Alkinyl.

Im Sinne der vorliegenden Erfindung ist (C₁-Cₙ)-Acyloxy eine Gruppe mit der allgemeinen Struktur R'-(C=O)O- mit insgesamt 1 bis n Kohlenstoffatomen, wobei R' ausgewählt wird aus der Gruppe bestehend aus H, (C₁-Cₙ₋₁) -Alkyl, (C₁-Cₙ₋₁) -Alkenyl, (C₆-Cₙ₋₁)-Aryl, (C₆-Cₙ₋₁) -Heteroaryl und (C₂-Cₙ₋₁) -Alkinyl.

Im Sinne der vorliegenden Erfindung ist (C₂-Cₙ)-Alkenyl definiert als lineare oder verzweigte (C₂-Cₙ)-Alkylgruppe mit 2 bis n Kohlenstoffatomen mit der Maßgabe, dass diese eine C-C-Doppelbindung aufweist.

Im Sinne der vorliegenden Erfindung ist (C₂-Cₙ)-Alkinyl definiert als lineare oder verzweigte (C₂-Cₙ)-Alkylgruppe mit 2 bis n Kohlenstoffatomen mit der Maßgabe, dass diese eine C-C-Dreifachbindung aufweist.

Im Sinne der vorliegenden Erfindung bezeichnet (C₆-Cₙ)-Aralkyl eine Gruppe, die sowohl eine Alkyl- als auch eine Arylgruppe enthält und in Summe 6 bis n Kohlenstoffatome aufweist. Die Aralkylgruppe kann über jedes ihrer Kohlenstoffatome an das diese Gruppe tragende Molekül gebunden sein. Eine (C₆-Cₙ)-Aralkylgruppe kann auch mit wenigstens einem Substituenten substitutiert sein, wobei die Substituenten unabhängig voneinander ausgewählt werden aus der Gruppe bestehend aus Halogen, (C₁-C₈)-Alkyl, (C₁-C₈)-Alkyloxy, -NH₂, -NO, -NO₂, NH(C₁-C₈)-Alkyl, -N((C₁-C₈)-Alkyl)₂, -OH, -CF₃, -CₙF₂ₙ₊₁ (wobei n eine ganze Zahl von 2 bis 5 ist), NH(C₁-C₈)-Acyl, -N((C₁-C₈)-Acyl)₂, (C₁-C₈)-Acyl, (C₁-C₈)-Acyloxy, -SO₂-(C₁-C₈)-Alkyl, -SO₂-(C₆-C₁₄)-Aryl, -SO-(C₁-C₈)-Alkyl, -SO-(C₆-C₁₄)-Aryl, -PO(O-{C₁-C₂₀}-Alkyl)₂, -PO(O-{C₆-C₁₄}-Aryl)₂, -PO(O-{C₁-C₂₀}-Alkyl)(O-{C₆-C₁₄}-Aryl), -PO({C₁-C₂₀}-Alkyl)₂, -PO({C₆-C₁₄}-Aryl)₂, -PO({C₁-C₂₀}-Alkyl) ({C₆-C₁₄}-Aryl).

Als Alkylierungsmittel können alle dem Fachmann für diesen Zweck in Frage kommenden Verbindungen ausgewählt werden, bevorzugt sind Alkylierungsmittel, die ausgewählt werden aus der Gruppe bestehend aus Alkylhalogeniden, Estern der Sulfonsäure, Estern der Fluorsulfonsäure, Estern der Trifluormethansulfonsäure, Estern der Chlorameisensäure, Oxonium-Salzen, Dialkylsulfaten und Diazomethan einzusetzen. Alkylierungsmittel, die ausgewählt werden aus der Gruppe bestehen aus (Me₃O)BF₄, (Et₃O)BF₄, (MeO)₂SO₂, (EtO)₂SO₂, ClCOOEt, MeI und EtI, sind dabei besonders bevorzugt.
Die Menge des Alkylierungsmittels kann vom Fachmann frei gewählt werden. Das Alkylierungsmittel wird jedoch bevorzugt in einem Mol-Verhältnis in einem Bereich von 1:1 bis 2:1 bezogen auf die zu reduzierende(n) Carbonyl-Gruppe(n) eingesetzt.

Als Hydrierkatalysator werden solche Hydrierkatalysatoren eingesetzt, die mindestens ein aktives Metall der Gruppe VII B und/oder VIII B des Periodensystems der Elemente, wobei Edelmetalle und Ni bevorzugt sind, insbesondere sind Ru, Rh, Pd, Pt, Re und Ni bevorzugt. Die Metalle können im Hydrierkatalysator entweder (a) als solche oder in Form von Oxiden oder (b) als Metallkomplexe vorliegen.

In Fall (a) kann das Metall oder Metalloxid entweder auf einem Träger aufgebracht oder als Partikel eingesetzt werden. Das Trägermaterial ist nicht beschränkt, üblicherweise werden gewöhnliche Träger wie Aluminiumoxid, Siliciumdioxid, Aluminiumoxid, Eisenoxid, Magnesiumoxid, Zirkoniumdioxid, Kohlenstoff oder ähnliche dem Fachmann auf dem Gebiet der Hydrierung bekannte Träger eingesetzt. Der Gehalt an Metall oder Metalloxid auf dem Träger wird in einem Bereich von 1 Gew.-% bis 25 Gew.-% bezogen auf das Gesamtgewicht des Katalysators gewählt. Bevorzugt wird ein Gehalt von 1 bis 5 Gew.-% Metall oder Metalloxid auf dem Träger gewählt. Beispiele für derartige Hydrierkatalysatoren sind Pt/C, Pd/C, Ru/C, Rh/C, Pd/CaCO₃, Pd/Al₂O₃, Ru/Al₂O₃, Rh/Al₂O₃, Pd/Re/C, Pt/Re/C, RuO₂.
In Fall (b) können die Metalle auch in Form von MetallKomplexen als Hydrierkatalysatoren eingesetzt werden. Beispiele hierfür sind Metall-Komplexe der Metalle Rh, Ir oder Ru, wie z.B. der Wilkinson-Katalysator ClRh(PPh₃)₃ oder [(dppb)Rh(cod)]BF₄, [Ir(PCy₃(C₅H₅N)(cod)]PF₆, [Cl₂Ru(PPh₃)₃] und [(dppb)Ru(metallyl)₂].
Bevorzugt wird der Hydrierkatalysator ausgewählt aus der Gruppe bestehend aus Pd/C, Pd/Al₂O₃, Pd/CaCO₃, Pt/C, Ru/Al₂O₃, Pd/Re/C, Pt/Re/C und [(dppb)Rh(cod)]BF₄. Besonders bevorzugt wird der Hydrierkatalysator ausgewählt aus der Gruppe bestehend aus 5%Pd/C, 5%Pd/Al₂O₃, 5%Pd/CaCO₃, 5%Pt/C, 5%Ru/Al₂O₃ und [(dppb)Rh(cod)]BF₄.
Die Menge des Hydrierkatalysators kann vom Fachmann frei gewählt werden, wobei das Mol-Verhältnis von Hydrierkatalysator zu Carbonsäureamid oder Carbonsäurediamid oder Di-, Tri- oder Polypeptid oder Peptidamid in einem Bereich von 1:10 bis 1:100.000 liegt. Weiter bevorzugt ist ein Bereich von 1:20 bis 1:10.000, besonders bevorzugt ist ein Bereich von 1:50 bis 1:2.000.

Prinzipiell ist der Fachmann frei in der Wahl des Lösungsmittels, das er in dem erfindungsgemäßen Verfahren einsetzen möchte. Aufgrund der Tatsache, dass die Ausgangsstoffe häufig in flüssiger Form vorliegen, kann insofern auf den Einsatz eines Lösungsmittels auch verzichtet werden. Wenn jedoch der Einsatz von Lösungsmitteln in dem erfindungsgemäßen Verfahren gewünscht ist, ist es vorteilhaft, solche Lösungsmittel heranzuziehen, die die eingesetzten Komponenten der Reaktion entsprechend gut lösen und sich im Übrigen gegenüber der erfindungsgemäßen Reaktion als inert erweisen. Als solche kommen polare und unpolare Lösungsmittel, insbesondere u.a. Kohlenwasserstoffe, Chlorkohlenwasserstoffe, Ether, Ester und Alkohole in Betracht. Bevorzugt sind dabei Alkane, Halogenalkane, einwertige und mehrwertige Alkohole, cyclische und acyclische Ether, und Ester.
Bevorzugte Lösungsmittel sind solche ausgewählt aus der Gruppe bestehend aus Hexan, Heptan, Octan, Dimethylglykolether (DMGE), 1,4-Dioxan, Methyl-tert-butylether (MTBE), Tetradydrofuran (THF), Essigsäureethylester, Essigsäureisopropyl-ester, Dibutylether, Dimethylcarbonat, Diethylcarbonat, Dipropylcarbonat, Methanol, Ethanol, Isopropanol, Butanol, Ethylenglycol, Dichlormethan, und 1,2-Dichlorethan. Besonders bevorzugt sind Methanol und Ethanol.

Der Wasserstoffdruck der Reaktion wird in einem Bereich von 0,1 bis 200 bar, vorzugsweise von 0,1 bis 100 bar, und besonders bevorzugt von 0,1 bis 60 bar eingestellt.

Die Temperatur, die während der Reaktion einzustellen ist, kann vom Fachmann bestimmt werden und liegt üblicherweise in einem Bereich von 0°C bis 120°C. Sie sollte so hoch sein, dass die anvisierte Reaktion in ausreichend schneller Zeit abläuft, doch möglichst so niedrig sein, dass das Nebenproduktspektrum bei der Hydrierung so gering wie möglich gehalten werden kann. Besonders bevorzugt wird eine Temperatur aus dem Bereich von 10°C bis 100°C eingestellt, ganz besonders bevorzugt wird eine Temperatur aus dem Bereich von 20°C bis 50°C eingestellt.

Bevorzugte Reaktionsbedingungen der Hydrierung können Tabelle 3 entnommen werden.

**Tabelle 3: Bevorzugte Reaktionsbedingungen bei der Hydrierung.**

| Druck [bar] | Temperatur [°C] | Hydrierkatalysator | Alkylierungsmittel, Mol-Verhältnis Carbonyl : Alkylierungsmi ttel 1:1-1:2 | Base | Lösungsmittel | Mol-Verhältnis Katalysator : Edukt | Edukt |
|---|---|---|---|---|---|---|---|
| | | | | | | | |
| | | | | | | | |
| 0,1-200 | 0°C-120°C | [b] | [e] | Gruppe (1) | ja | 1:20-1:10.000 | [g] |
| 0,1-200 | 0°C-120°C | [b] | [e] | Gruppe (1) | nein | 1:20-1:10.000 | [g] |
| 0,1-200 | 10°C-100°C | [b] | [e] | Gruppe (1) | ja | 1:20-1:10.000 | [g] |
| 0,1-200 | 10°C-100°C | [b] | [e] | Gruppe (1) | nein | 1:20-1:10.000 | [g] |
| 0,1-200 | 20°C-50°C | [b] | [e] | Gruppe (1) | ja | 1:20-1:10.000 | [g] |
| 0,1-200 | 20°C-50°C | [b] | [e] | Gruppe (1) | nein | 1:20-1:10.000 | [g] |
| | | | | | | | |
| | | | | | | | |
| 0,1-100 | 0°C-120°C | [b] | [e] | Gruppe (1) | ja | 1:20-1:10.000 | [g] |
| 0,1-100 | 0°C-120°C | [b] | [e] | Gruppe (1) | nein | 1:20-1:10.000 | [g] |
| 0,1-100 | 10°C-100°C | [b] | [e] | Gruppe (1) | ja | 1:20-1:10.000 | [g] |
| 0,1-100 | 10°C-100°C | [b] | [e] | Gruppe (1) | nein | 1:20-1:10.000 | [g] |
| 0,1-100 | 20°C-50°C | [b] | [e] | Gruppe (1) | ja | 1:20-1:10.000 | [g] |
| 0,1-100 | 20°C-50°C | [b] | [e] | Gruppe (1) | nein | 1:20-1:10.000 | [g] |
| 0,1-60 | 0°C-120°C | [b] | [e] | Gruppe (1) | ja | 1:20-1:10.000 | [g] |
| 0,1-60 | 0°C-120°C | [b] | [e] | Gruppe (1) | nein | 1:20-1:10.000 | [g] |
| 0,1-60 | 10°C-100°C | [b] | [e] | Gruppe (1) | ja | 1:20-1:10.000 | [g] |
| 0,1-60 | 10°C-100°C | [b] | [e] | Gruppe (1) | nein | 1:20-1:10.000 | [g] |
| 0,1-60 | 20°C-50°C | [b] | [e] | Gruppe (1) | ja | 1:20-1:10.000 | [g] |
| 0,1-60 | 20°C-50°C | [b] | [e] | Gruppe (1) | nein | 1:20-1:10.000 | [g] |
| 0,1-200 | 0°C-120°C | [b] | [e] | Gruppe (2) | ja | 1:20-1:10.000 | [g] |
| 0,1-200 | 0°C-120°C | [b] | [e] | Gruppe (2) | nein | 1:20-1:10.000 | [g] |
| 0,1-200 | 10°C-100°C | [b] | [e] | Gruppe (2) | ja | 1:20-1:10.000 | [g] |
| 0,1-200 | 10°C-100°C | [b] | [e] | Gruppe (2) | nein | 1:20-1:10.000 | [g] |
| 0,1-200 | 20°C-50°C | [b] | [e] | Gruppe (2) | ja | 1:20-1:10.000 | [g] |
| 0,1-200 | 20°C-50°C | [b] | [e] | Gruppe (2) | nein | 1:20-1:10.000 | [g] |
| | | | | | | | |
| | | | | | | | |
| 0,1-100 | 0°C-120°C | [b] | [e] | Gruppe (2) | ja | 1:20-1:10.000 | [g] |
| 0,1-100 | 0°C-120°C | [b] | [e] | Gruppe (2) | nein | 1:20-1:10.000 | [g] |
| 0,1-100 | 10°C-100°C | [b] | [e] | Gruppe (2) | ja | 1:20-1:10.000 | [g] |
| 0,1-100 | 10°C-100°C | [b] | [e] | Gruppe (2) | nein | 1:20-1:10.000 | [g] |
| 0,1-100 | 20°C-50°C | [b] | [e] | Gruppe (2) | ja | 1:20-1:10.000 | [g] |
| 0,1-100 | 20°C-50°C | [b] | [e] | Gruppe (2) | nein | 1:20-1:10.000 | [g] |
| | | | | | | | |
| | | | | | | | |
| 0,1-60 | 0°C-120°C | [b] | [e] | Gruppe (2) | ja | 1:20-1:10.000 | [g] |
| 0,1-60 | 0°C-120°C | [b] | [e] | Gruppe (2) | nein | 1:20-1:10.000 | [g] |
| 0,1-60 | 10°C-100°C | [b] | [e] | Gruppe (2) | ja | 1:20-1:10.000 | [g] |
| 0,1-60 | 10°C-100°C | [b] | [e] | Gruppe (2) | nein | 1:20-1:10.000 | [g] |
| 0,1-60 | 20°C-50°C | [b] | [e] | Gruppe (2) | ja | 1:20-1:10.000 | [g] |
| 0,1-60 | 20°C-50°C | [b] | [e] | Gruppe (2) | nein | 1:20-1:10.000 | [g] |
| | | | | | | | |
| | | | | | | | |
| 0,1-200 | 0°C-120°C | [c] | [e] | Gruppe (1) | ja | 1:20-1:10.000 | [g] |
| 0,1-200 | 0°C-120°C | [c] | [e] | Gruppe (1) | nein | 1:20-1:10.000 | [g] |
| 0,1-200 | 10°C-100°C | [c] | [e] | Gruppe (1) | ja | 1:20-1:10.000 | [g] |
| 0,1-200 | 10°C-100°C | [c] | [e] | Gruppe (1) | nein | 1:20-1:10.000 | [g] |
| 0,1-200 | 20°C-50°C | [c] | [e] | Gruppe (1) | ja | 1:20-1:10.000 | [g] |
| 0,1-200 | 20°C-50°C | [c] | [e] | Gruppe (1) | nein | 1:20-1:10.000 | [g] |
| 0,1-100 | 0°C-120°C | [c] | [e] | Gruppe (1) | ja | 1:20-1:10.000 | [g] |
| 0,1-100 | 0°C-120°C | [c] | [e] | Gruppe (1) | nein | 1:20-1:10.000 | [g] |
| 0,1-100 | 10°C-100°C | [c] | [e] | Gruppe (1) | ja | 1:20-1:10.000 | [g] |
| 0,1-100 | 10°C-100°C | [c] | [e] | Gruppe (1) | nein | 1:20-1:10.000 | [g] |
| 0,1-100 | 20°C-50°C | [c] | [e] | Gruppe (1) | ja | 1:20-1:10.000 | [g] |
| 0,1-100 | 20°C-50°C | [c] | [e] | Gruppe (1) | nein | 1:20-1:10.000 | [g] |
| 0,1-60 | 0°C-120°C | [c] | [e] | Gruppe (1) | ja | 1:20-1:10.000 | [g] |
| 0,1-60 | 0°C-120°C | [c] | [e] | Gruppe (1) | nein | 1:20-1:10.000 | [g] |
| 0,1-60 | 10°C-100°C | [c] | [e] | Gruppe (1) | ja | 1:20-1:10.000 | [g] |
| 0,1-60 | 10°C-100°C | [c] | [e] | Gruppe (1) | nein | 1:20-1:10.000 | [g] |
| 0,1-60 | 20°C-50°C | [c] | [e] | Gruppe (1) | ja | 1:20-1:10.000 | [g] |
| 0,1-60 | 20°C-50°C | [c] | [e] | Gruppe (1) | nein | 1:20-1:10.000 | [g] |
| | | | | | | | |
| | | | | | | | |
| 0,1-200 | 0°C-120°C | [c] | [e] | Gruppe (2) | ja | 1:20-1:10.000 | [g] |
| 0,1-200 | 0°C-120°C | [c] | [e] | Gruppe (2) | nein | 1:20-1:10.000 | [g] |
| 0,1-200 | 10°C-100°C | [c] | [e] | Gruppe (2) | ja | 1:20-1:10.000 | [g] |
| 0,1-200 | 10°C-100°C | [c] | [e] | Gruppe (2) | nein | 1:20-1:10.000 | [g] |
| 0,1-200 | 20°C-50°C | [c] | [e] | Gruppe (2) | ja | 1:20-1:10.000 | [g] |
| 0,1-200 | 20°C-50°C | [c] | [e] | Gruppe (2) | nein | 1:20-1:10.000 | [g] |
| | | | | | | | |
| | | | | | | | |
| 0,1-100 | 0°C-120°C | [c] | [e] | Gruppe (2) | ja | 1:20-1:10.000 | [g] |
| 0,1-100 | 0°C-120°C | [c] | [e] | Gruppe (2) | nein | 1:20-1:10.000 | [g] |
| 0,1-100 | 10°C-100°C | [c] | [e] | Gruppe (2) | ja | 1:20-1:10.000 | [g] |
| 0,1-100 | 10°C-100°C | [c] | [e] | Gruppe (2) | nein | 1:20-1:10.000 | [g] |
| 0,1-100 | 20°C-50°C | [c] | [e] | Gruppe (2) | ja | 1:20-1:10.000 | [g] |
| 0,1-100 | 20°C-50°C | [c] | [e] | Gruppe (2) | nein | 1:20-1:10.000 | [g] |
| | | | | | | | |
| | | | | | | | |
| 0,1-60 | 0°C-120°C | [c] | [e] | Gruppe (2) | ja | 1:20-1:10.000 | [g] |
| 0,1-60 | 0°C-120°C | [c] | [e] | Gruppe (2) | nein | 1:20-1:10.000 | [g] |
| 0,1-60 | 10°C-100°C | [c] | [e] | Gruppe (2) | ja | 1:20-1:10.000 | [g] |
| 0,1-60 | 10°C-100°C | [c] | [e] | Gruppe (2) | nein | 1:20-1:10.000 | [g] |
| 0,1-60 | 20°C-50°C | [c] | [e] | Gruppe (2) | ja | 1:20-1:10.000 | [g] |
| 0,1-60 | 20°C-50°C | [c] | [e] | Gruppe (2) | nein | 1:20-1:10.000 | [g] |
| 0,1-200 | 0°C-120°C | [d] | [e] | Gruppe (1) | ja | 1:20-1:10.000 | [g] |
| 0,1-200 | 0°C-120°C | [d] | [e] | Gruppe (1) | nein | 1:20-1:10.000 | [g] |
| 0,1-200 | 10°C-100°C | [d] | [e] | Gruppe (1) | ja | 1:20-1:10.000 | [g] |
| 0,1-200 | 10°C-100°C | [d] | [e] | Gruppe (1) | nein | 1:20-1:10.000 | [g] |
| 0,1-200 | 20°C-50°C | [d] | [e] | Gruppe (1) | ja | 1:20-1:10.000 | [g] |
| 0,1-200 | 20°C-50°C | [d] | [e] | Gruppe (1) | nein | 1:20-1:10.000 | [g] |
| 0,1-100 | 0°C-120°C | [d] | [e] | Gruppe (1) | ja | 1:20-1:10.000 | [g] |
| 0,1-100 | 0°C-120°C | [d] | [e] | Gruppe (1) | nein | 1:20-1:10.000 | [g] |
| 0,1-100 | 10°C-100°C | [d] | [e] | Gruppe (1) | ja | 1:20-1:10.000 | [g] |
| 0,1-100 | 10°C-100°C | [d] | [e] | Gruppe (1) | nein | 1:20-1:10.000 | [g] |
| 0,1-100 | 20°C-50°C | [d] | [e] | Gruppe (1) | ja | 1:20-1:10.000 | [g] |
| 0,1-100 | 20°C-50°C | [d] | [e] | Gruppe (1) | nein | 1:20-1:10.000 | [g] |
| | | | | | | | |
| | | | | | | | |
| 0,1-60 | 0°C-120°C | [d] | [e] | Gruppe (1) | ja | 1:20-1:10.000 | [g] |
| 0,1-60 | 0°C-120°C | [d] | [e] | Gruppe (1) | nein | 1:20-1:10.000 | [g] |
| 0,1-60 | 10°C-100°C | [d] | [e] | Gruppe (1) | ja | 1:20-1:10.000 | [g] |
| 0,1-60 | 10°C-100°C | [d] | [e] | Gruppe (1) | nein | 1:20-1:10.000 | [g] |
| 0,1-60 | 20°C-50°C | [d] | [e] | Gruppe (1) | ja | 1:20-1:10.000 | [g] |
| 0,1-60 | 20°C-50°C | [d] | [e] | Gruppe (1) | nein | 1:20-1:10.000 | [g] |
| | | | | | | | |
| | | | | | | | |
| 0,1-200 | 0°C-120°C | [d] | [e] | Gruppe (2) | ja | 1:20-1:10.000 | [g] |
| 0,1-200 | 0°C-120°C | [d] | [e] | Gruppe (2) | nein | 1:20-1:10.000 | [g] |
| 0,1-200 | 10°C-100°C | [d] | [e] | Gruppe (2) | ja | 1:20-1:10.000 | [g] |
| 0,1-200 | 10°C-100°C | [d] | [e] | Gruppe (2) | nein | 1:20-1:10.000 | [g] |
| 0,1-200 | 20°C-50°C | [d] | [e] | Gruppe (2) | ja | 1:20-1:10.000 | [g] |
| 0,1-200 | 20°C-50°C | [d] | [e] | Gruppe (2) | nein | 1:20-1:10.000 | [g] |
| | | | | | | | |
| | | | | | | | |
| 0,1-100 | 0°C-120°C | [d] | [e] | Gruppe (2) | ja | 1:20-1:10.000 | [g] |
| 0,1-100 | 0°C-120°C | [d] | [e] | Gruppe (2) | nein | 1:20-1:10.000 | [g] |
| 0,1-100 | 10°C-100°C | [d] | [e] | Gruppe (2) | ja | 1:20-1:10.000 | [g] |
| 0,1-100 | 10°C-100°C | [d] | [e] | Gruppe (2) | nein | 1:20-1:10.000 | [g] |
| 0,1-100 | 20°C-50°C | [d] | [e] | Gruppe (2) | ja | 1:20-1:10.000 | [g] |
| 0,1-100 | 20°C-50°C | [d] | [e] | Gruppe (2) | nein | 1:20-1:10.000 | [g] |
| 0,1-60 | 0°C-120°C | [d] | [e] | Gruppe (2) | ja | 1:20-1:10.000 | [g] |
| 0,1-60 | 0°C-120°C | [d] | [e] | Gruppe (2) | nein | 1:20-1:10.000 | [g] |
| 0,1-60 | 10°C-100°C | [d] | [e] | Gruppe (2) | ja | 1:20-1:10.000 | [g] |
| 0,1-60 | 10°C-100°C | [d] | [e] | Gruppe (2) | nein | 1:20-1:10.000 | [g] |
| 0,1-60 | 20°C-50°C | [d] | [e] | Gruppe (2) | ja | 1:20-1:10.000 | [g] |
| 0,1-60 | 20°C-50°C | [d] | [e] | Gruppe (2) | nein | 1:20-1:10.000 | [g] |
| 0,1-200 | 0°C-120°C | [d] | [f] | Gruppe (1) | ja | 1:20-1:10.000 | [g] |
| 0,1-200 | 0°C-120°C | [d] | [f] | Gruppe (1) | nein | 1:20-1:10.000 | [g] |
| 0,1-200 | 10°C-100°C | [d] | [f] | Gruppe (1) | ja | 1:20-1:10.000 | [g] |
| 0,1-200 | 10°C-100°C | [d] | [f] | Gruppe (1) | nein | 1:20-1:10.000 | [g] |
| 0,1-200 | 20°C-50°C | [d] | [f] | Gruppe (1) | ja | 1:20-1:10.000 | [g] |
| 0,1-200 | 20°C-50°C | [d] | [f] | Gruppe (1) | nein | 1:20-1:10.000 | [g] |
| | | | | | | | |
| | | | | | | | |
| 0,1-100 | 0°C-120°C | [d] | [f] | Gruppe (1) | ja | 1:20-1:10.000 | [g] |
| 0,1-100 | 0°C-120°C | [d] | [f] | Gruppe (1) | nein | 1:20-1:10.000 | [g] |
| 0,1-100 | 10°C-100°C | [d] | [f] | Gruppe (1) | ja | 1:20-1:10.000 | [g] |
| 0,1-100 | 10°C-100°C | [d] | [f] | Gruppe (1) | nein | 1:20-1:10.000 | [g] |
| 0,1-100 | 20°C-50°C | [d] | [f] | Gruppe (1) | ja | 1:20-1:10.000 | [g] |
| 0,1-100 | 20°C-50°C | [d] | [f] | Gruppe (1) | nein | 1:20-1:10.000 | [g] |
| | | | | | | | |
| | | | | | | | |
| 0,1-60 | 0°C-120°C | [d] | [f] | Gruppe (1) | ja | 1:20-1:10.000 | [g] |
| 0,1-60 | 0°C-120°C | [d] | [f] | Gruppe (1) | nein | 1:20-1:10.000 | [g] |
| 0,1-60 | 10°C-100°C | [d] | [f] | Gruppe (1) | ja | 1:20-1:10.000 | [g] |
| 0,1-60 | 10°C-100°C | [d] | [f] | Gruppe (1) | nein | 1:20-1:10.000 | [g] |
| 0,1-60 | 20°C-50°C | [d] | [f] | Gruppe (1) | ja | 1:20-1:10.000 | [g] |
| 0,1-60 | 20°C-50°C | [d] | [f] | Gruppe (1) | nein | 1:20-1:10.000 | [g] |
| | | | | | | | |
| | | | | | | | |
| 0,1-200 | 0°C-120°C | [d] | [f] | Gruppe (2) | ja | 1:20-1:10.000 | [g] |
| 0,1-200 | 0°C-120°C | [d] | [f] | Gruppe (2) | nein | 1:20-1:10.000 | [g] |
| 0,1-200 | 10°C-100°C | [d] | [f] | Gruppe (2) | ja | 1:20-1:10.000 | [g] |
| 0,1-200 | 10°C-100°C | [d] | [f] | Gruppe (2) | nein | 1:20-1:10.000 | [g] |
| 0,1-200 | 20°C-50°C | [d] | [f] | Gruppe (2) | ja | 1:20-1:10.000 | [g] |
| 0,1-200 | 20°C-50°C | [d] | [f] | Gruppe (2) | nein | 1:20-1:10.000 | [g] |
| 0,1-100 | 0°C-120°C | [d] | [f] | Gruppe (2) | ja | 1:20-1:10.000 | [g] |
| 0,1-100 | 0°C-120°C | [d] | [f] | Gruppe (2) | nein | 1:20-1:10.000 | [g] |
| 0,1-100 | 10°C-100°C | [d] | [f] | Gruppe (2) | ja | 1:20-1:10.000 | [g] |
| 0,1-100 | 10°C-100°C | [d] | [f] | Gruppe (2) | nein | 1:20-1:10.000 | [g] |
| 0,1-100 | 20°C-50°C | [d] | [f] | Gruppe (2) | ja | 1:20-1:10.000 | [g] |
| 0,1-100 | 20°C-50°C | [d] | [f] | Gruppe (2) | nein | 1:20-1:10.000 | [g] |
| 0,1-60 | 0°C-120°C | [d] | [f] | Gruppe (2) | ja | 1:20-1:10.000 | [g] |
| 0,1-60 | 0°C-120°C | [d] | [f] | Gruppe (2) | nein | 1:20-1:10.000 | [g] |
| 0,1-60 | 10°C-100°C | [d] | [f] | Gruppe (2) | ja | 1:20-1:10.000 | [g] |
| 0,1-60 | 10°C-100°C | [d] | [f] | Gruppe (2) | nein | 1:20-1:10.000 | [g] |
| 0,1-60 | 20°C-50°C | [d] | [f] | Gruppe (2) | ja | 1:20-1:10.000 | [g] |
| 0,1-60 | 20°C-50°C | [d] | [f] | Gruppe (2) | nein | 1:20-1:10.000 | [g] |
| | | | | | | | |
| | | | | | | | |
| 0,1-200 | 0°C-120°C | [d] | [e] | Gruppe (1) | ja | 1:20-1:10.000 | [h] |
| 0,1-200 | 0°C-120°C | [d] | [e] | Gruppe (1) | nein | 1:20-1:10.000 | [h] |
| 0,1-200 | 10°C-100°C | [d] | [e] | Gruppe (1) | ja | 1:20-1:10.000 | [h] |
| 0,1-200 | 10°C-100°C | [d] | [e] | Gruppe (1) | nein | 1:20-1:10.000 | [h] |
| 0,1-200 | 20°C-50°C | [d] | [e] | Gruppe (1) | ja | 1:20-1:10.000 | [h] |
| 0,1-200 | 20°C-50°C | [d] | [e] | Gruppe (1) | nein | 1:20-1:10.000 | [h] |
| | | | | | | | |
| | | | | | | | |
| 0,1-100 | 0°C-120°C | [d] | [e] | Gruppe (1) | ja | 1:20-1:10.000 | [h] |
| 0,1-100 | 0°C-120°C | [d] | [e] | Gruppe (1) | nein | 1:20-1:10.000 | [h] |
| 0,1-100 | 10°C-100°C | [d] | [e] | Gruppe (1) | ja | 1:20-1:10.000 | [h] |
| 0,1-100 | 10°C-100°C | [d] | [e] | Gruppe (1) | nein | 1:20-1:10.000 | [h] |
| 0,1-100 | 20°C-50°C | [d] | [e] | Gruppe (1) | ja | 1:20-1:10.000 | [h] |
| 0,1-100 | 20°C-50°C | [d] | [e] | Gruppe (1) | nein | 1:20-1:10.000 | [h] |
| | | | | | | | |
| | | | | | | | |
| 0,1-60 | 0°C-120°C | [d] | [e] | Gruppe (1) | ja | 1:20-1:10.000 | [h] |
| 0,1-60 | 0°C-120°C | [d] | [e] | Gruppe (1) | nein | 1:20-1:10.000 | [h] |
| 0,1-60 | 10°C-100°C | [d] | [e] | Gruppe (1) | ja | 1:20-1:10.000 | [h] |
| 0,1-60 | 10°C-100°C | [d] | [e] | Gruppe (1) | nein | 1:20-1:10.000 | [h] |
| 0,1-60 | 20°C-50°C | [d] | [e] | Gruppe (1) | ja | 1:20-1:10.000 | [h] |
| 0,1-60 | 20°C-50°C | [d] | [e] | Gruppe (1) | nein | 1:20-1:10.000 | [h] |
| 0,1-200 | 0°C-120°C | [d] | [e] | Gruppe (2) | ja | 1:20-1:10.000 | [h] |
| 0,1-200 | 0°C-120°C | [d] | [e] | Gruppe (2) | nein | 1:20-1:10.000 | [h] |
| 0,1-200 | 10°C-100°C | [d] | [e] | Gruppe (2) | ja | 1:20-1:10.000 | [h] |
| 0,1-200 | 10°C-100°C | [d] | [e] | Gruppe (2) | nein | 1:20-1:10.000 | [h] |
| 0,1-200 | 20°C-50°C | [d] | [e] | Gruppe (2) | ja | 1:20-1:10.000 | [h] |
| 0,1-200 | 20°C-50°C | [d] | [e] | Gruppe (2) | nein | 1:20-1:10.000 | [h] |
| 0,1-100 | 0°C-120°C | [d] | [e] | Gruppe (2) | ja | 1:20-1:10.000 | [h] |
| 0,1-100 | 0°C-120°C | [d] | [e] | Gruppe (2) | nein | 1:20-1:10.000 | [h] |
| 0,1-100 | 10°C-100°C | [d] | [e] | Gruppe (2) | ja | 1:20-1:10.000 | [h] |
| 0,1-100 | 10°C-100°C | [d] | [e] | Gruppe (2) | nein | 1:20-1:10.000 | [h] |
| 0,1-100 | 20°C-50°C | [d] | [e] | Gruppe (2) | ja | 1:20-1:10.000 | [h] |
| 0,1-100 | 20°C-50°C | [d] | [e] | Gruppe (2) | nein | 1:20-1:10.000 | [h] |
| | | | | | | | |
| | | | | | | | |
| 0,1-60 | 0°C-120°C | [d] | [e] | Gruppe (2) | ja | 1:20-1:10.000 | [h] |
| 0,1-60 | 0°C-120°C | [d] | [e] | Gruppe (2) | nein | 1:20-1:10.000 | [h] |
| 0,1-60 | 10°C-100°C | [d] | [e] | Gruppe (2) | ja | 1:20-1:10.000 | [h] |
| 0,1-60 | 10°C-100°C | [d] | [e] | Gruppe (2) | nein | 1:20-1:10.000 | [h] |
| 0,1-60 | 20°C-50°C | [d] | [e] | Gruppe (2) | ja | 1:20-1:10.000 | [h] |
| 0,1-60 | 20°C-50°C | [d] | [e] | Gruppe (2) | nein | 1:20-1:10.000 | [h] |
| | | | | | | | |
| | | | | | | | |
| 0,1-200 | 0°C-120°C | [d] | [f] | Gruppe (1) | ja | 1:20-1:10.000 | [h] |
| 0,1-200 | 0°C-120°C | [d] | [f] | Gruppe (1) | nein | 1:20-1:10.000 | [h] |
| 0,1-200 | 10°C-100°C | [d] | [f] | Gruppe (1) | ja | 1:20-1:10.000 | [h] |
| 0,1-200 | 10°C-100°C | [d] | [f] | Gruppe (1) | nein | 1:20-1:10.000 | [h] |
| 0,1-200 | 20°C-50°C | [d] | [f] | Gruppe (1) | ja | 1:20-1:10.000 | [h] |
| 0,1-200 | 20°C-50°C | [d] | [f] | Gruppe (1) | nein | 1:20-1:10.000 | [h] |
| | | | | | | | |
| | | | | | | | |
| 0,1-100 | 0°C-120°C | [d] | [f] | Gruppe (1) | ja | 1:20-1:10.000 | [h] |
| 0,1-100 | 0°C-120°C | [d] | [f] | Gruppe (1) | nein | 1:20-1:10.000 | [h] |
| 0,1-100 | 10°C-100°C | [d] | [f] | Gruppe (1) | ja | 1:20-1:10.000 | [h] |
| 0,1-100 | 10°C-100°C | [d] | [f] | Gruppe (1) | nein | 1:20-1:10.000 | [h] |
| 0,1-100 | 20°C-50°C | [d] | [f] | Gruppe (1) | ja | 1:20-1:10.000 | [h] |
| 0,1-100 | 20°C-50°C | [d] | [f] | Gruppe (1) | nein | 1:20-1:10.000 | [h] |
| 0,1-60 | 0°C-120°C | [d] | [f] | Gruppe (1) | ja | 1:20-1:10.000 | [h] |
| 0,1-60 | 0°C-120°C | [d] | [f] | Gruppe (1) | nein | 1:20-1:10.000 | [h] |
| 0,1-60 | 10°C-100°C | [d] | [f] | Gruppe (1) | ja | 1:20-1:10.000 | [h] |
| 0,1-60 | 10°C-100°C | [d] | [f] | Gruppe (1) | nein | 1:20-1:10.000 | [h] |
| 0,1-60 | 20°C-50°C | [d] | [f] | Gruppe (1) | ja | 1:20-1:10.000 | [h] |
| 0,1-60 | 20°C-50°C | [d] | [f] | Gruppe (1) | nein | 1:20-1:10.000 | [h] |
| 0,1-200 | 0°C-120°C | [d] | [f] | Gruppe (2) | ja | 1:20-1:10.000 | [h] |
| 0,1-200 | 0°C-120°C | [d] | [f] | Gruppe (2) | nein | 1:20-1:10.000 | [h] |
| 0,1-200 | 10°C-100°C | [d] | [f] | Gruppe (2) | ja | 1:20-1:10.000 | [h] |
| 0,1-200 | 10°C-100°C | [d] | [f] | Gruppe (2) | nein | 1:20-1:10.000 | [h] |
| 0,1-200 | 20°C-50°C | [d] | [f] | Gruppe (2) | ja | 1:20-1:10.000 | [h] |
| 0,1-200 | 20°C-50°C | [d] | [f] | Gruppe (2) | nein | 1:20-1:10.000 | [h] |
| | | | | | | | |
| | | | | | | | |
| 0,1-100 | 0°C-120°C | [d] | [f] | Gruppe (2) | ja | 1:20-1:10.000 | [h] |
| 0,1-100 | 0°C-120°C | [d] | [f] | Gruppe (2) | nein | 1:20-1:10.000 | [h] |
| 0,1-100 | 10°C-100°C | [d] | [f] | Gruppe (2) | ja | 1:20-1:10.000 | [h] |
| 0,1-100 | 10°C-100°C | [d] | [f] | Gruppe (2) | nein | 1:20-1:10.000 | [h] |
| 0,1-100 | 20°C-50°C | [d] | [f] | Gruppe (2) | ja | 1:20-1:10.000 | [h] |
| 0,1-100 | 20°C-50°C | [d] | [f] | Gruppe (2) | nein | 1:20-1:10.000 | [h] |
| | | | | | | | |
| | | | | | | | |
| 0,1-60 | 0°C-120°C | [d] | [f] | Gruppe (2) | ja | 1:20-1:10.000 | [h] |
| 0,1-60 | 0°C-120°C | [d] | [f] | Gruppe (2) | nein | 1:20-1:10.000 | [h] |
| 0,1-60 | 10°C-100°C | [d] | [f] | Gruppe (2) | ja | 1:20-1:10.000 | [h] |
| 0,1-60 | 10°C-100°C | [d] | [f] | Gruppe (2) | nein | 1:20-1:10.000 | [h] |
| 0,1-60 | 20°C-50°C | [d] | [f] | Gruppe (2) | ja | 1:20-1:10.000 | [h] |
| 0,1-60 | 20°C-50°C | [d] | [f] | Gruppe (2) | nein | 1:20-1:10.000 | [h] |
| | | | | | | | |
| | | | | | | | |
| 0,1-200 | 0°C-120°C | [d] | [e] | Gruppe (1) | ja | 1:20-1:10.000 | [i] |
| 0,1-200 | 0°C-120°C | [d] | [e] | Gruppe (1) | nein | 1:20-1:10.000 | [i] |
| 0,1-200 | 10°C-100°C | [d] | [e] | Gruppe (1) | ja | 1:20-1:10.000 | [i] |
| 0,1-200 | 10°C-100°C | [d] | [e] | Gruppe (1) | nein | 1:20-1:10.000 | [i] |
| 0,1-200 | 20°C-50°C | [d] | [e] | Gruppe (1) | ja | 1:20-1:10.000 | [i] |
| 0,1-200 | 20°C-50°C | [d] | [e] | Gruppe (1) | nein | 1:20-1:10.000 | [i] |
| 0,1-100 | 0°C-120°C | [d] | [e] | Gruppe (1) | ja | 1:20-1:10.000 | [i] |
| 0,1-100 | 0°C-120°C | [d] | [e] | Gruppe (1) | nein | 1:20-1:10.000 | [i] |
| 0,1-100 | 10°C-100°C | [d] | [e] | Gruppe (1) | ja | 1:20-1:10.000 | [i] |
| 0,1-100 | 10°C-100°C | [d] | [e] | Gruppe (1) | nein | 1:20-1:10.000 | [i] |
| 0,1-100 | 20°C-50°C | [d] | [e] | Gruppe (1) | ja | 1:20-1:10.000 | [i] |
| 0,1-100 | 20°C-50°C | [d] | [e] | Gruppe (1) | nein | 1:20-1:10.000 | [i] |
| 0,1-60 | 0°C-120°C | [d] | [e] | Gruppe (1) | ja | 1:20-1:10.000 | [i] |
| 0,1-60 | 0°C-120°C | [d] | [e] | Gruppe (1) | nein | 1:20-1:10.000 | [i] |
| 0,1-60 | 10°C-100°C | [d] | [e] | Gruppe (1) | ja | 1:20-1:10.000 | [i] |
| 0,1-60 | 10°C-100°C | [d] | [e] | Gruppe (1) | nein | 1:20-1:10.000 | [i] |
| 0,1-60 | 20°C-50°C | [d] | [e] | Gruppe (1) | ja | 1:20-1:10.000 | [i] |
| 0,1-60 | 20°C-50°C | [d] | [e] | Gruppe (1) | nein | 1:20-1:10.000 | [i] |
| | | | | | | | |
| | | | | | | | |
| 0,1-200 | 0°C-120°C | [d] | [e] | Gruppe (2) | ja | 1:20-1:10.000 | [i] |
| 0,1-200 | 0°C-120°C | [d] | [e] | Gruppe (2) | nein | 1:20-1:10.000 | [i] |
| 0,1-200 | 10°C-100°C | [d] | [e] | Gruppe (2) | ja | 1:20-1:10.000 | [i] |
| 0,1-200 | 10°C-100°C | [d] | [e] | Gruppe (2) | nein | 1:20-1:10.000 | [i] |
| 0,1-200 | 20°C-50°C | [d] | [e] | Gruppe (2) | ja | 1:20-1:10.000 | [i] |
| 0,1-200 | 20°C-50°C | [d] | [e] | Gruppe (2) | nein | 1:20-1:10.000 | [i] |
| | | | | | | | |
| | | | | | | | |
| 0,1-100 | 0°C-120°C | [d] | [e] | Gruppe (2) | ja | 1:20-1:10.000 | [i] |
| 0,1-100 | 0°C-120°C | [d] | [e] | Gruppe (2) | nein | 1:20-1:10.000 | [i] |
| 0,1-100 | 10°C-100°C | [d] | [e] | Gruppe (2) | ja | 1:20-1:10.000 | [i] |
| 0,1-100 | 10°C-100°C | [d] | [e] | Gruppe (2) | nein | 1:20-1:10.000 | [i] |
| 0,1-100 | 20°C-50°C | [d] | [e] | Gruppe (2) | ja | 1:20-1:10.000 | [i] |
| 0,1-100 | 20°C-50°C | [d] | [e] | Gruppe (2) | nein | 1:20-1:10.000 | [i] |
| | | | | | | | |
| | | | | | | | |
| 0,1-60 | 0°C-120°C | [d] | [e] | Gruppe (2) | ja | 1:20-1:10.000 | [i] |
| 0,1-60 | 0°C-120°C | [d] | [e] | Gruppe (2) | nein | 1:20-1:10.000 | [i] |
| 0,1-60 | 10°C-100°C | [d] | [e] | Gruppe (2) | ja | 1:20-1:10.000 | [i] |
| 0,1-60 | 10°C-100°C | [d] | [e] | Gruppe (2) | nein | 1:20-1:10.000 | [i] |
| 0,1-60 | 20°C-50°C | [d] | [e] | Gruppe (2) | ja | 1:20-1:10.000 | [i] |
| 0,1-60 | 20°C-50°C | [d] | [e] | Gruppe (2) | nein | 1:20-1:10.000 | [i] |
| 0,1-200 | 0°C-120°C | [d] | [f] | Gruppe (1) | ja | 1:20-1:10.000 | [i] |
| 0,1-200 | 0°C-120°C | [d] | [f] | Gruppe (1) | nein | 1:20-1:10.000 | [i] |
| 0,1-200 | 10°C-100°C | [d] | [f] | Gruppe (1) | ja | 1:20-1:10.000 | [i] |
| 0,1-200 | 10°C-100°C | [d] | [f] | Gruppe (1) | nein | 1:20-1:10.000 | [i] |
| 0,1-200 | 20°C-50°C | [d] | [f] | Gruppe (1) | ja | 1:20-1:10.000 | [i] |
| 0,1-200 | 20°C-50°C | [d] | [f] | Gruppe (1) | nein | 1:20-1:10.000 | [i] |
| 0,1-100 | 0°C-120°C | [d] | [f] | Gruppe (1) | ja | 1:20-1:10.000 | [i] |
| 0,1-100 | 0°C-120°C | [d] | [f] | Gruppe (1) | nein | 1:20-1:10.000 | [i] |
| 0,1-100 | 10°C-100°C | [d] | [f] | Gruppe (1) | ja | 1:20-1:10.000 | [i] |
| 0,1-100 | 10°C-100°C | [d] | [f] | Gruppe (1) | nein | 1:20-1:10.000 | [i] |
| 0,1-100 | 20°C-50°C | [d] | [f] | Gruppe (1) | ja | 1:20-1:10.000 | [i] |
| 0,1-100 | 20°C-50°C | [d] | [f] | Gruppe (1) | nein | 1:20-1:10.000 | [i] |
| | | | | | | | |
| | | | | | | | |
| 0,1-60 | 0°C-120°C | [d] | [f] | Gruppe (1) | ja | 1:20-1:10.000 | [i] |
| 0,1-60 | 0°C-120°C | [d] | [f] | Gruppe (1) | nein | 1:20-1:10.000 | [i] |
| 0,1-60 | 10°C-100°C | [d] | [f] | Gruppe (1) | ja | 1:20-1:10.000 | [i] |
| 0,1-60 | 10°C-100°C | [d] | [f] | Gruppe (1) | nein | 1:20-1:10.000 | [i] |
| 0,1-60 | 20°C-50°C | [d] | [f] | Gruppe (1) | ja | 1:20-1:10.000 | [i] |
| 0,1-60 | 20°C-50°C | [d] | [f] | Gruppe (1) | nein | 1:20-1:10.000 | [i] |
| | | | | | | | |
| | | | | | | | |
| 0,1-200 | 0°C-120°C | [d] | [f] | Gruppe (2) | ja | 1:20-1:10.000 | [i] |
| 0,1-200 | 0°C-120°C | [d] | [f] | Gruppe (2) | nein | 1:20-1:10.000 | [i] |
| 0,1-200 | 10°C-100°C | [d] | [f] | Gruppe (2) | ja | 1:20-1:10.000 | [i] |
| 0,1-200 | 10°C-100°C | [d] | [f] | Gruppe (2) | nein | 1:20-1:10.000 | [i] |
| 0,1-200 | 20°C-50°C | [d] | [f] | Gruppe (2) | ja | 1:20-1:10.000 | [i] |
| 0,1-200 | 20°C-50°C | [d] | [f] | Gruppe (2) | nein | 1:20-1:10.000 | [i] |
| | | | | | | | |
| | | | | | | | |
| 0,1-100 | 0°C-120°C | [d] | [f] | Gruppe (2) | ja | 1:20-1:10.000 | [i] |
| 0,1-100 | 0°C-120°C | [d] | [f] | Gruppe (2) | nein | 1:20-1:10.000 | [i] |
| 0,1-100 | 10°C-100°C | [d] | [f] | Gruppe (2) | ja | 1:20-1:10.000 | [i] |
| 0,1-100 | 10°C-100°C | [d] | [f] | Gruppe (2) | nein | 1:20-1:10.000 | [i] |
| 0,1-100 | 20°C-50°C | [d] | [f] | Gruppe (2) | ja | 1:20-1:10.000 | [i] |
| 0,1-100 | 20°C-50°C | [d] | [f] | Gruppe (2) | nein | 1:20-1:10.000 | [i] |
| 0,1-60 | 0°C-120°C | [d] | [f] | Gruppe (2) | ja | 1:20-1:10.000 | [i] |
| 0,1-60 | 0°C-120°C | [d] | [f] | Gruppe (2) | nein | 1:20-1:10.000 | [i] |
| 0,1-60 | 10°C-100°C | [d] | [f] | Gruppe (2) | ja | 1:20-1:10.000 | [i] |
| 0,1-60 | 10°C-100°C | [d] | [f] | Gruppe (2) | nein | 1:20-1:10.000 | [i] |
| 0,1-60 | 20°C-50°C | [d] | [f] | Gruppe (2) | ja | 1:20-1:10.000 | [i] |
| 0,1-60 | 20°C-50°C | [d] | [f] | Gruppe (2) | nein | 1:20-1:10.000 | [i] |
| 0,1-200 | 0°C-120°C | [b] | [e] | Gruppe (1) | ja | 1:50-1:2.000 | [g] |
| 0,1-200 | 0°C-120°C | [b] | [e] | Gruppe (1) | nein | 1:50-1:2.000 | [g] |
| 0,1-200 | 10°C-100°C | [b] | [e] | Gruppe (1) | ja | 1:50-1:2.000 | [g] |
| 0,1-200 | 10°C-100°C | [b] | [e] | Gruppe (1) | nein | 1:50-1:2.000 | [g] |
| 0,1-200 | 20°C-50°C | [b] | [e] | Gruppe (1) | ja | 1:50-1:2.000 | [g] |
| 0,1-200 | 20°C-50°C | [b] | [e] | Gruppe (1) | nein | 1:50-1:2.000 | [g] |
| | | | | | | | |
| | | | | | | | |
| 0,1-100 | 0°C-120°C | [b] | [e] | Gruppe (1) | ja | 1:50-1:2.000 | [g] |
| 0,1-100 | 0°C-120°C | [b] | [e] | Gruppe (1) | nein | 1:50-1:2.000 | [g] |
| 0,1-100 | 10°C-100°C | [b] | [e] | Gruppe (1) | ja | 1:50-1:2.000 | [g] |
| 0,1-100 | 10°C-100°C | [b] | [e] | Gruppe (1) | nein | 1:50-1:2.000 | [g] |
| 0,1-100 | 20°C-50°C | [b] | [e] | Gruppe (1) | ja | 1:50-1:2.000 | [g] |
| 0,1-100 | 20°C-50°C | [b] | [e] | Gruppe (1) | nein | 1:50-1:2.000 | [g] |
| | | | | | | | |
| | | | | | | | |
| 0,1-60 | 0°C-120°C | [b] | [e] | Gruppe (1) | ja | 1:50-1:2.000 | [g] |
| 0,1-60 | 0°C-120°C | [b] | [e] | Gruppe (1) | nein | 1:50-1:2.000 | [g] |
| 0,1-60 | 10°C-100°C | [b] | [e] | Gruppe (1) | ja | 1:50-1:2.000 | [g] |
| 0,1-60 | 10°C-100°C | [b] | [e] | Gruppe (1) | nein | 1:50-1:2.000 | [g] |
| 0,1-60 | 20°C-50°C | [b] | [e] | Gruppe (1) | ja | 1:50-1:2.000 | [g] |
| 0,1-60 | 20°C-50°C | [b] | [e] | Gruppe (1) | nein | 1:50-1:2.000 | [g] |
| | | | | | | | |
| | | | | | | | |
| 0,1-200 | 0°C-120°C | [b] | [e] | Gruppe (2) | ja | 1:50-1:2.000 | [g] |
| 0,1-200 | 0°C-120°C | [b] | [e] | Gruppe (2) | nein | 1:50-1:2.000 | [g] |
| 0,1-200 | 10°C-100°C | [b] | [e] | Gruppe (2) | ja | 1:50-1:2.000 | [g] |
| 0,1-200 | 10°C-100°C | [b] | [e] | Gruppe (2) | nein | 1:50-1:2.000 | [g] |
| 0,1-200 | 20°C-50°C | [b] | [e] | Gruppe (2) | ja | 1:50-1:2.000 | [g] |
| 0,1-200 | 20°C-50°C | [b] | [e] | Gruppe (2) | nein | 1:50-1:2.000 | [g] |
| 0,1-100 | 0°C-120°C | [b] | [e] | Gruppe (2) | ja | 1:50-1:2.000 | [g] |
| 0,1-100 | 0°C-120°C | [b] | [e] | Gruppe (2) | nein | 1:50-1:2.000 | [g] |
| 0,1-100 | 10°C-100°C | [b] | [e] | Gruppe (2) | ja | 1:50-1:2.000 | [g] |
| 0,1-100 | 10°C-100°C | [b] | [e] | Gruppe (2) | nein | 1:50-1:2.000 | [g] |
| 0,1-100 | 20°C-50°C | [b] | [e] | Gruppe (2) | ja | 1:50-1:2.000 | [g] |
| 0,1-100 | 20°C-50°C | [b] | [e] | Gruppe (2) | nein | 1:50-1:2.000 | [g] |
| 0,1-60 | 0°C-120°C | [b] | [e] | Gruppe (2) | ja | 1:50-1:2.000 | [g] |
| 0,1-60 | 0°C-120°C | [b] | [e] | Gruppe (2) | nein | 1:50-1:2.000 | [g] |
| 0,1-60 | 10°C-100°C | [b] | [e] | Gruppe (2) | ja | 1:50-1:2.000 | [g] |
| 0,1-60 | 10°C-100°C | [b] | [e] | Gruppe (2) | nein | 1:50-1:2.000 | [g] |
| 0,1-60 | 20°C-50°C | [b] | [e] | Gruppe (2) | ja | 1:50-1:2.000 | [g] |
| 0,1-60 | 20°C-50°C | [b] | [e] | Gruppe (2) | nein | 1:50-1:2.000 | [g] |
| | | | | | | | |
| | | | | | | | |
| 0,1-200 | 0°C-120°C | [c] | [e] | Gruppe (1) | ja | 1:50-1:2.000 | [g] |
| 0,1-200 | 0°C-120°C | [c] | [e] | Gruppe (1) | nein | 1:50-1:2.000 | [g] |
| 0,1-200 | 10°C-100°C | [c] | [e] | Gruppe (1) | ja | 1:50-1:2.000 | [g] |
| 0,1-200 | 10°C-100°C | [c] | [e] | Gruppe (1) | nein | 1:50-1:2.000 | [g] |
| 0,1-200 | 20°C-50°C | [c] | [e] | Gruppe (1) | ja | 1:50-1:2.000 | [g] |
| 0,1-200 | 20°C-50°C | [c] | [e] | Gruppe (1) | nein | 1:50-1:2.000 | [g] |
| | | | | | | | |
| | | | | | | | |
| 0,1-100 | 0°C-120°C | [c] | [e] | Gruppe (1) | ja | 1:50-1:2.000 | [g] |
| 0,1-100 | 0°C-120°C | [c] | [e] | Gruppe (1) | nein | 1:50-1:2.000 | [g] |
| 0,1-100 | 10°C-100°C | [c] | [e] | Gruppe (1) | ja | 1:50-1:2.000 | [g] |
| 0,1-100 | 10°C-100°C | [c] | [e] | Gruppe (1) | nein | 1:50-1:2.000 | [g] |
| 0,1-100 | 20°C-50°C | [c] | [e] | Gruppe (1) | ja | 1:50-1:2.000 | [g] |
| 0,1-100 | 20°C-50°C | [c] | [e] | Gruppe (1) | nein | 1:50-1:2.000 | [g] |
| | | | | | | | |
| | | | | | | | |
| 0,1-60 | 0°C-120°C | [c] | [e] | Gruppe (1) | ja | 1:50-1:2.000 | [g] |
| 0,1-60 | 0°C-120°C | [c] | [e] | Gruppe (1) | nein | 1:50-1:2.000 | [g] |
| 0,1-60 | 10°C-100°C | [c] | [e] | Gruppe (1) | ja | 1:50-1:2.000 | [g] |
| 0,1-60 | 10°C-100°C | [c] | [e] | Gruppe (1) | nein | 1:50-1:2.000 | [g] |
| 0,1-60 | 20°C-50°C | [c] | [e] | Gruppe (1) | ja | 1:50-1:2.000 | [g] |
| 0,1-60 | 20°C-50°C | [c] | [e] | Gruppe (1) | nein | 1:50-1:2.000 | [g] |
| 0,1-200 | 0°C-120°C | [c] | [e] | Gruppe (2) | ja | 1:50-1:2.000 | [g] |
| 0,1-200 | 0°C-120°C | [c] | [e] | Gruppe (2) | nein | 1:50-1:2.000 | [g] |
| 0,1-200 | 10°C-100°C | [c] | [e] | Gruppe (2) | ja | 1:50-1:2.000 | [g] |
| 0,1-200 | 10°C-100°C | [c] | [e] | Gruppe (2) | nein | 1:50-1:2.000 | [g] |
| 0,1-200 | 20°C-50°C | [c] | [e] | Gruppe (2) | ja | 1:50-1:2.000 | [g] |
| 0,1-200 | 20°C-50°C | [c] | [e] | Gruppe (2) | nein | 1:50-1:2.000 | [g] |
| 0,1-100 | 0°C-120°C | [c] | [e] | Gruppe (2) | ja | 1:50-1:2.000 | [g] |
| 0,1-100 | 0°C-120°C | [c] | [e] | Gruppe (2) | nein | 1:50-1:2.000 | [g] |
| 0,1-100 | 10°C-100°C | [c] | [e] | Gruppe (2) | ja | 1:50-1:2.000 | [g] |
| 0,1-100 | 10°C-100°C | [c] | [e] | Gruppe (2) | nein | 1:50-1:2.000 | [g] |
| 0,1-100 | 20°C-50°C | [c] | [e] | Gruppe (2) | ja | 1:50-1:2.000 | [g] |
| 0,1-100 | 20°C-50°C | [c] | [e] | Gruppe (2) | nein | 1:50-1:2.000 | [g] |
| | | | | | | | |
| | | | | | | | |
| 0,1-60 | 0°C-120°C | [c] | [e] | Gruppe (2) | ja | 1:50-1:2.000 | [g] |
| 0,1-60 | 0°C-120°C | [c] | [e] | Gruppe (2) | nein | 1:50-1:2.000 | [g] |
| 0,1-60 | 10°C-100°C | [c] | [e] | Gruppe (2) | ja | 1:50-1:2.000 | [g] |
| 0,1-60 | 10°C-100°C | [c] | [e] | Gruppe (2) | nein | 1:50-1:2.000 | [g] |
| 0,1-60 | 20°C-50°C | [c] | [e] | Gruppe (2) | ja | 1:50-1:2.000 | [g] |
| 0,1-60 | 20°C-50°C | [c] | [e] | Gruppe (2) | nein | 1:50-1:2.000 | [g] |
| | | | | | | | |
| | | | | | | | |
| 0,1-200 | 0°C-120°C | [d] | [e] | Gruppe (1) | ja | 1:50-1:2.000 | [g] |
| 0,1-200 | 0°C-120°C | [d] | [e] | Gruppe (1) | nein | 1:50-1:2.000 | [g] |
| 0,1-200 | 10°C-100°C | [d] | [e] | Gruppe (1) | ja | 1:50-1:2.000 | [g] |
| 0,1-200 | 10°C-100°C | [d] | [e] | Gruppe (1) | nein | 1:50-1:2.000 | [g] |
| 0,1-200 | 20°C-50°C | [d] | [e] | Gruppe (1) | ja | 1:50-1:2.000 | [g] |
| 0,1-200 | 20°C-50°C | [d] | [e] | Gruppe (1) | nein | 1:50-1:2.000 | [g] |
| | | | | | | | |
| | | | | | | | |
| 0,1-100 | 0°C-120°C | [d] | [e] | Gruppe (1) | ja | 1:50-1:2.000 | [g] |
| 0,1-100 | 0°C-120°C | [d] | [e] | Gruppe (1) | nein | 1:50-1:2.000 | [g] |
| 0,1-100 | 10°C-100°C | [d] | [e] | Gruppe (1) | ja | 1:50-1:2.000 | [g] |
| 0,1-100 | 10°C-100°C | [d] | [e] | Gruppe (1) | nein | 1:50-1:2.000 | [g] |
| 0,1-100 | 20°C-50°C | [d] | [e] | Gruppe (1) | ja | 1:50-1:2.000 | [g] |
| 0,1-100 | 20°C-50°C | [d] | [e] | Gruppe (1) | nein | 1:50-1:2.000 | [g] |
| 0,1-60 | 0°C-120°C | [d] | [e] | Gruppe (1) | ja | 1:50-1:2.000 | [g] |
| 0,1-60 | 0°C-120°C | [d] | [e] | Gruppe (1) | nein | 1:50-1:2.000 | [g] |
| 0,1-60 | 10°C-100°C | [d] | [e] | Gruppe (1) | ja | 1:50-1:2.000 | [g] |
| 0,1-60 | 10°C-100°C | [d] | [e] | Gruppe (1) | nein | 1:50-1:2.000 | [g] |
| 0,1-60 | 20°C-50°C | [d] | [e] | Gruppe (1) | ja | 1:50-1:2.000 | [g] |
| 0,1-60 | 20°C-50°C | [d] | [e] | Gruppe (1) | nein | 1:50-1:2.000 | [g] |
| 0,1-200 | 0°C-120°C | [d] | [e] | Gruppe (2) | ja | 1:50-1:2.000 | [g] |
| 0,1-200 | 0°C-120°C | [d] | [e] | Gruppe (2) | nein | 1:50-1:2.000 | [g] |
| 0,1-200 | 10°C-100°C | [d] | [e] | Gruppe (2) | ja | 1:50-1:2.000 | [g] |
| 0,1-200 | 10°C-100°C | [d] | [e] | Gruppe (2) | nein | 1:50-1:2.000 | [g] |
| 0,1-200 | 20°C-50°C | [d] | [e] | Gruppe (2) | ja | 1:50-1:2.000 | [g] |
| 0,1-200 | 20°C-50°C | [d] | [e] | Gruppe (2) | nein | 1:50-1:2.000 | [g] |
| | | | | | | | |
| | | | | | | | |
| 0,1-100 | 0°C-120°C | [d] | [e] | Gruppe (2) | ja | 1:50-1:2.000 | [g] |
| 0,1-100 | 0°C-120°C | [d] | [e] | Gruppe (2) | nein | 1:50-1:2.000 | [g] |
| 0,1-100 | 10°C-100°C | [d] | [e] | Gruppe (2) | ja | 1:50-1:2.000 | [g] |
| 0,1-100 | 10°C-100°C | [d] | [e] | Gruppe (2) | nein | 1:50-1:2.000 | [g] |
| 0,1-100 | 20°C-50°C | [d] | [e] | Gruppe (2) | ja | 1:50-1:2.000 | [g] |
| 0,1-100 | 20°C-50°C | [d] | [e] | Gruppe (2) | nein | 1:50-1:2.000 | [g] |
| | | | | | | | |
| | | | | | | | |
| 0,1-60 | 0°C-120°C | [d] | [e] | Gruppe (2) | ja | 1:50-1:2.000 | [g] |
| 0,1-60 | 0°C-120°C | [d] | [e] | Gruppe (2) | nein | 1:50-1:2.000 | [g] |
| 0,1-60 | 10°C-100°C | [d] | [e] | Gruppe (2) | ja | 1:50-1:2.000 | [g] |
| 0,1-60 | 10°C-100°C | [d] | [e] | Gruppe (2) | nein | 1:50-1:2.000 | [g] |
| 0,1-60 | 20°C-50°C | [d] | [e] | Gruppe (2) | ja | 1:50-1:2.000 | [g] |
| 0,1-60 | 20°C-50°C | [d] | [e] | Gruppe (2) | nein | 1:50-1:2.000 | [g] |
| | | | | | | | |
| | | | | | | | |
| 0,1-200 | 0°C-120°C | [d] | [f] | Gruppe (1) | ja | 1:50-1:2.000 | [g] |
| 0,1-200 | 0°C-120°C | [d] | [f] | Gruppe (1) | nein | 1:50-1:2.000 | [g] |
| 0,1-200 | 10°C-100°C | [d] | [f] | Gruppe (1) | ja | 1:50-1:2.000 | [g] |
| 0,1-200 | 10°C-100°C | [d] | [f] | Gruppe (1) | nein | 1:50-1:2.000 | [g] |
| 0,1-200 | 20°C-50°C | [d] | [f] | Gruppe (1) | ja | 1:50-1:2.000 | [g] |
| 0,1-200 | 20°C-50°C | [d] | [f] | Gruppe (1) | nein | 1:50-1:2.000 | [g] |
| 0,1-100 | 0°C-120°C | [d] | [f] | Gruppe (1) | ja | 1:50-1:2.000 | [g] |
| 0,1-100 | 0°C-120°C | [d] | [f] | Gruppe (1) | nein | 1:50-1:2.000 | [g] |
| 0,1-100 | 10°C-100°C | [d] | [f] | Gruppe (1) | ja | 1:50-1:2.000 | [g] |
| 0,1-100 | 10°C-100°C | [d] | [f] | Gruppe (1) | nein | 1:50-1:2.000 | [g] |
| 0,1-100 | 20°C-50°C | [d] | [f] | Gruppe (1) | ja | 1:50-1:2.000 | [g] |
| 0,1-100 | 20°C-50°C | [d] | [f] | Gruppe (1) | nein | 1:50-1:2.000 | [g] |
| 0,1-60 | 0°C-120°C | [d] | [f] | Gruppe (1) | ja | 1:50-1:2.000 | [g] |
| 0,1-60 | 0°C-120°C | [d] | [f] | Gruppe (1) | nein | 1:50-1:2.000 | [g] |
| 0,1-60 | 10°C-100°C | [d] | [f] | Gruppe (1) | ja | 1:50-1:2.000 | [g] |
| 0,1-60 | 10°C-100°C | [d] | [f] | Gruppe (1) | nein | 1:50-1:2.000 | [g] |
| 0,1-60 | 20°C-50°C | [d] | [f] | Gruppe (1) | ja | 1:50-1:2.000 | [g] |
| 0,1-60 | 20°C-50°C | [d] | [f] | Gruppe (1) | nein | 1:50-1:2.000 | [g] |
| | | | | | | | |
| | | | | | | | |
| 0,1-200 | 0°C-120°C | [d] | [f] | Gruppe (2) | ja | 1:50-1:2.000 | [g] |
| 0,1-200 | 0°C-120°C | [d] | [f] | Gruppe (2) | nein | 1:50-1:2.000 | [g] |
| 0,1-200 | 10°C-100°C | [d] | [f] | Gruppe (2) | ja | 1:50-1:2.000 | [g] |
| 0,1-200 | 10°C-100°C | [d] | [f] | Gruppe (2) | nein | 1:50-1:2.000 | [g] |
| 0,1-200 | 20°C-50°C | [d] | [f] | Gruppe (2) | ja | 1:50-1:2.000 | [g] |
| 0,1-200 | 20°C-50°C | [d] | [f] | Gruppe (2) | nein | 1:50-1:2.000 | [g] |
| | | | | | | | |
| | | | | | | | |
| 0,1-100 | 0°C-120°C | [d] | [f] | Gruppe (2) | ja | 1:50-1:2.000 | [g] |
| 0,1-100 | 0°C-120°C | [d] | [f] | Gruppe (2) | nein | 1:50-1:2.000 | [g] |
| 0,1-100 | 10°C-100°C | [d] | [f] | Gruppe (2) | ja | 1:50-1:2.000 | [g] |
| 0,1-100 | 10°C-100°C | [d] | [f] | Gruppe (2) | nein | 1:50-1:2.000 | [g] |
| 0,1-100 | 20°C-50°C | [d] | [f] | Gruppe (2) | ja | 1:50-1:2.000 | [g] |
| 0,1-100 | 20°C-50°C | [d] | [f] | Gruppe (2) | nein | 1:50-1:2.000 | [g] |
| | | | | | | | |
| | | | | | | | |
| 0,1-60 | 0°C-120°C | [d] | [f] | Gruppe (2) | ja | 1:50-1:2.000 | [g] |
| 0,1-60 | 0°C-120°C | [d] | [f] | Gruppe (2) | nein | 1:50-1:2.000 | [g] |
| 0,1-60 | 10°C-100°C | [d] | [f] | Gruppe (2) | ja | 1:50-1:2.000 | [g] |
| 0,1-60 | 10°C-100°C | [d] | [f] | Gruppe (2) | nein | 1:50-1:2.000 | [g] |
| 0,1-60 | 20°C-50°C | [d] | [f] | Gruppe (2) | ja | 1:50-1:2.000 | [g] |
| 0,1-60 | 20°C-50°C | [d] | [f] | Gruppe (2) | nein | 1:50-1:2.000 | [g] |
| 0,1-200 | 0°C-120°C | [d] | [e] | Gruppe (1) | ja | 1:50-1:2.000 | [h] |
| 0,1-200 | 0°C-120°C | [d] | [e] | Gruppe (1) | nein | 1:50-1:2.000 | [h] |
| 0,1-200 | 10°C-100°C | [d] | [e] | Gruppe (1) | ja | 1:50-1:2.000 | [h] |
| 0,1-200 | 10°C-100°C | [d] | [e] | Gruppe (1) | nein | 1:50-1:2.000 | [h] |
| 0,1-200 | 20°C-50°C | [d] | [e] | Gruppe (1) | ja | 1:50-1:2.000 | [h] |
| 0,1-200 | 20°C-50°C | [d] | [e] | Gruppe (1) | nein | 1:50-1:2.000 | [h] |
| 0,1-100 | 0°C-120°C | [d] | [e] | Gruppe (1) | ja | 1:50-1:2.000 | [h] |
| 0,1-100 | 0°C-120°C | [d] | [e] | Gruppe (1) | nein | 1:50-1:2.000 | [h] |
| 0,1-100 | 10°C-100°C | [d] | [e] | Gruppe (1) | ja | 1:50-1:2.000 | [h] |
| 0,1-100 | 10°C-100°C | [d] | [e] | Gruppe (1) | nein | 1:50-1:2.000 | [h] |
| 0,1-100 | 20°C-50°C | [d] | [e] | Gruppe (1) | ja | 1:50-1:2.000 | [h] |
| 0,1-100 | 20°C-50°C | [d] | [e] | Gruppe (1) | nein | 1:50-1:2.000 | [h] |
| | | | | | | | |
| | | | | | | | |
| 0,1-60 | 0°C-120°C | [d] | [e] | Gruppe (1) | ja | 1:50-1:2.000 | [h] |
| 0,1-60 | 0°C-120°C | [d] | [e] | Gruppe (1) | nein | 1:50-1:2.000 | [h] |
| 0,1-60 | 10°C-100°C | [d] | [e] | Gruppe (1) | ja | 1:50-1:2.000 | [h] |
| 0,1-60 | 10°C-100°C | [d] | [e] | Gruppe (1) | nein | 1:50-1:2.000 | [h] |
| 0,1-60 | 20°C-50°C | [d] | [e] | Gruppe (1) | ja | 1:50-1:2.000 | [h] |
| 0,1-60 | 20°C-50°C | [d] | [e] | Gruppe (1) | nein | 1:50-1:2.000 | [h] |
| | | | | | | | |
| | | | | | | | |
| 0,1-200 | 0°C-120°C | [d] | [e] | Gruppe (2) | ja | 1:50-1:2.000 | [h] |
| 0,1-200 | 0°C-120°C | [d] | [e] | Gruppe (2) | nein | 1:50-1:2.000 | [h] |
| 0,1-200 | 10°C-100°C | [d] | [e] | Gruppe (2) | ja | 1:50-1:2.000 | [h] |
| 0,1-200 | 10°C-100°C | [d] | [e] | Gruppe (2) | nein | 1:50-1:2.000 | [h] |
| 0,1-200 | 20°C-50°C | [d] | [e] | Gruppe (2) | ja | 1:50-1:2.000 | [h] |
| 0,1-200 | 20°C-50°C | [d] | [e] | Gruppe (2) | nein | 1:50-1:2.000 | [h] |
| | | | | | | | |
| | | | | | | | |
| 0,1-100 | 0°C-120°C | [d] | [e] | Gruppe (2) | ja | 1:50-1:2.000 | [h] |
| 0,1-100 | 0°C-120°C | [d] | [e] | Gruppe (2) | nein | 1:50-1:2.000 | [h] |
| 0,1-100 | 10°C-100°C | [d] | [e] | Gruppe (2) | ja | 1:50-1:2.000 | [h] |
| 0,1-100 | 10°C-100°C | [d] | [e] | Gruppe (2) | nein | 1:50-1:2.000 | [h] |
| 0,1-100 | 20°C-50°C | [d] | [e] | Gruppe (2) | ja | 1:50-1:2.000 | [h] |
| 0,1-100 | 20°C-50°C | [d] | [e] | Gruppe (2) | nein | 1:50-1:2.000 | [h] |
| 0,1-60 | 0°C-120°C | [d] | [e] | Gruppe (2) | ja | 1:50-1:2.000 | [h] |
| 0,1-60 | 0°C-120°C | [d] | [e] | Gruppe (2) | nein | 1:50-1:2.000 | [h] |
| 0,1-60 | 10°C-100°C | [d] | [e] | Gruppe (2) | ja | 1:50-1:2.000 | [h] |
| 0,1-60 | 10°C-100°C | [d] | [e] | Gruppe (2) | nein | 1:50-1:2.000 | [h] |
| 0,1-60 | 20°C-50°C | [d] | [e] | Gruppe (2) | ja | 1:50-1:2.000 | [h] |
| 0,1-60 | 20°C-50°C | [d] | [e] | Gruppe (2) | nein | 1:50-1:2.000 | [h] |
| 0,1-200 | 0°C-120°C | [d] | [f] | Gruppe (1) | ja | 1:50-1:2.000 | [h] |
| 0,1-200 | 0°C-120°C | [d] | [f] | Gruppe (1) | nein | 1:50-1:2.000 | [h] |
| 0,1-200 | 10°C-100°C | [d] | [f] | Gruppe (1) | ja | 1:50-1:2.000 | [h] |
| 0,1-200 | 10°C-100°C | [d] | [f] | Gruppe (1) | nein | 1:50-1:2.000 | [h] |
| 0,1-200 | 20°C-50°C | [d] | [f] | Gruppe (1) | ja | 1:50-1:2.000 | [h] |
| 0,1-200 | 20°C-50°C | [d] | [f] | Gruppe (1) | nein | 1:50-1:2.000 | [h] |
| | | | | | | | |
| | | | | | | | |
| 0,1-100 | 0°C-120°C | [d] | [f] | Gruppe (1) | ja | 1:50-1:2.000 | [h] |
| 0,1-100 | 0°C-120°C | [d] | [f] | Gruppe (1) | nein | 1:50-1:2.000 | [h] |
| 0,1-100 | 10°C-100°C | [d] | [f] | Gruppe (1) | ja | 1:50-1:2.000 | [h] |
| 0,1-100 | 10°C-100°C | [d] | [f] | Gruppe (1) | nein | 1:50-1:2.000 | [h] |
| 0,1-100 | 20°C-50°C | [d] | [f] | Gruppe (1) | ja | 1:50-1:2.000 | [h] |
| 0,1-100 | 20°C-50°C | [d] | [f] | Gruppe (1) | nein | 1:50-1:2.000 | [h] |
| | | | | | | | |
| | | | | | | | |
| 0,1-60 | 0°C-120°C | [d] | [f] | Gruppe (1) | ja | 1:50-1:2.000 | [h] |
| 0,1-60 | 0°C-120°C | [d] | [f] | Gruppe (1) | nein | 1:50-1:2.000 | [h] |
| 0,1-60 | 10°C-100°C | [d] | [f] | Gruppe (1) | ja | 1:50-1:2.000 | [h] |
| 0,1-60 | 10°C-100°C | [d] | [f] | Gruppe (1) | nein | 1:50-1:2.000 | [h] |
| 0,1-60 | 20°C-50°C | [d] | [f] | Gruppe (1) | ja | 1:50-1:2.000 | [h] |
| 0,1-60 | 20°C-50°C | [d] | [f] | Gruppe (1) | nein | 1:50-1:2.000 | [h] |
| | | | | | | | |
| | | | | | | | |
| 0,1-200 | 0°C-120°C | [d] | [f] | Gruppe (2) | ja | 1:50-1:2.000 | [h] |
| 0,1-200 | 0°C-120°C | [d] | [f] | Gruppe (2) | nein | 1:50-1:2.000 | [h] |
| 0,1-200 | 10°C-100°C | [d] | [f] | Gruppe (2) | ja | 1:50-1:2.000 | [h] |
| 0,1-200 | 10°C-100°C | [d] | [f] | Gruppe (2) | nein | 1:50-1:2.000 | [h] |
| 0,1-200 | 20°C-50°C | [d] | [f] | Gruppe (2) | ja | 1:50-1:2.000 | [h] |
| 0,1-200 | 20°C-50°C | [d] | [f] | Gruppe (2) | nein | 1:50-1:2.000 | [h] |
| 0,1-100 | 0°C-120°C | [d] | [f] | Gruppe (2) | ja | 1:50-1:2.000 | [h] |
| 0,1-100 | 0°C-120°C | [d] | [f] | Gruppe (2) | nein | 1:50-1:2.000 | [h] |
| 0,1-100 | 10°C-100°C | [d] | [f] | Gruppe (2) | ja | 1:50-1:2.000 | [h] |
| 0,1-100 | 10°C-100°C | [d] | [f] | Gruppe (2) | nein | 1:50-1:2.000 | [h] |
| 0,1-100 | 20°C-50°C | [d] | [f] | Gruppe (2) | ja | 1:50-1:2.000 | [h] |
| 0,1-100 | 20°C-50°C | [d] | [f] | Gruppe (2) | nein | 1:50-1:2.000 | [h] |
| 0,1-60 | 0°C-120°C | [d] | [f] | Gruppe (2) | ja | 1:50-1:2.000 | [h] |
| 0,1-60 | 0°C-120°C | [d] | [f] | Gruppe (2) | nein | 1:50-1:2.000 | [h] |
| 0,1-60 | 10°C-100°C | [d] | [f] | Gruppe (2) | ja | 1:50-1:2.000 | [h] |
| 0,1-60 | 10°C-100°C | [d] | [f] | Gruppe (2) | nein | 1:50-1:2.000 | [h] |
| 0,1-60 | 20°C-50°C | [d] | [f] | Gruppe (2) | ja | 1:50-1:2.000 | [h] |
| 0,1-60 | 20°C-50°C | [d] | [f] | Gruppe (2) | nein | 1:50-1:2.000 | [h] |
| | | | | | | | |
| | | | | | | | |
| 0,1-200 | 0°C-120°C | [d] | [e] | Gruppe (1) | ja | 1:50-1:2.000 | [i] |
| 0,1-200 | 0°C-120°C | [d] | [e] | Gruppe (1) | nein | 1:50-1:2.000 | [i] |
| 0,1-200 | 10°C-100°C | [d] | [e] | Gruppe (1) | ja | 1:50-1:2.000 | [i] |
| 0,1-200 | 10°C-100°C | [d] | [e] | Gruppe (1) | nein | 1:50-1:2.000 | [i] |
| 0,1-200 | 20°C-50°C | [d] | [e] | Gruppe (1) | ja | 1:50-1:2.000 | [i] |
| 0,1-200 | 20°C-50°C | [d] | [e] | Gruppe (1) | nein | 1:50-1:2.000 | [i] |
| | | | | | | | |
| | | | | | | | |
| 0,1-100 | 0°C-120°C | [d] | [e] | Gruppe (1) | ja | 1:50-1:2.000 | [i] |
| 0,1-100 | 0°C-120°C | [d] | [e] | Gruppe (1) | nein | 1:50-1:2.000 | [i] |
| 0,1-100 | 10°C-100°C | [d] | [e] | Gruppe (1) | ja | 1:50-1:2.000 | [i] |
| 0,1-100 | 10°C-100°C | [d] | [e] | Gruppe (1) | nein | 1:50-1:2.000 | [i] |
| 0,1-100 | 20°C-50°C | [d] | [e] | Gruppe (1) | ja | 1:50-1:2.000 | [i] |
| 0,1-100 | 20°C-50°C | [d] | [e] | Gruppe (1) | nein | 1:50-1:2.000 | [i] |
| | | | | | | | |
| | | | | | | | |
| 0,1-60 | 0°C-120°C | [d] | [e] | Gruppe (1) | ja | 1:50-1:2.000 | [i] |
| 0,1-60 | 0°C-120°C | [d] | [e] | Gruppe (1) | nein | 1:50-1:2.000 | [i] |
| 0,1-60 | 10°C-100°C | [d] | [e] | Gruppe (1) | ja | 1:50-1:2.000 | [i] |
| 0,1-60 | 10°C-100°C | [d] | [e] | Gruppe (1) | nein | 1:50-1:2.000 | [i] |
| 0,1-60 | 20°C-50°C | [d] | [e] | Gruppe (1) | ja | 1:50-1:2.000 | [i] |
| 0,1-60 | 20°C-50°C | [d] | [e] | Gruppe (1) | nein | 1:50-1:2.000 | [i] |
| 0,1-200 | 0°C-120°C | [d] | [e] | Gruppe (2) | ja | 1:50-1:2.000 | [i] |
| 0,1-200 | 0°C-120°C | [d] | [e] | Gruppe (2) | nein | 1:50-1:2.000 | [i] |
| 0,1-200 | 10°C-100°C | [d] | [e] | Gruppe (2) | ja | 1:50-1:2.000 | [i] |
| 0,1-200 | 10°C-100°C | [d] | [e] | Gruppe (2) | nein | 1:50-1:2.000 | [i] |
| 0,1-200 | 20°C-50°C | [d] | [e] | Gruppe (2) | ja | 1:50-1:2.000 | [i] |
| 0,1-200 | 20°C-50°C | [d] | [e] | Gruppe (2) | nein | 1:50-1:2.000 | [i] |
| 0,1-100 | 0°C-120°C | [d] | [e] | Gruppe (2) | ja | 1:50-1:2.000 | [i] |
| 0,1-100 | 0°C-120°C | [d] | [e] | Gruppe (2) | nein | 1:50-1:2.000 | [i] |
| 0,1-100 | 10°C-100°C | [d] | [e] | Gruppe (2) | ja | 1:50-1:2.000 | [i] |
| 0,1-100 | 10°C-100°C | [d] | [e] | Gruppe (2) | nein | 1:50-1:2.000 | [i] |
| 0,1-100 | 20°C-50°C | [d] | [e] | Gruppe (2) | ja | 1:50-1:2.000 | [i] |
| 0,1-100 | 20°C-50°C | [d] | [e] | Gruppe (2) | nein | 1:50-1:2.000 | [i] |
| | | | | | | | |
| | | | | | | | |
| 0,1-60 | 0°C-120°C | [d] | [e] | Gruppe (2) | ja | 1:50-1:2.000 | [i] |
| 0,1-60 | 0°C-120°C | [d] | [e] | Gruppe (2) | nein | 1:50-1:2.000 | [i] |
| 0,1-60 | 10°C-100°C | [d] | [e] | Gruppe (2) | ja | 1:50-1:2.000 | [i] |
| 0,1-60 | 10°C-100°C | [d] | [e] | Gruppe (2) | nein | 1:50-1:2.000 | [i] |
| 0,1-60 | 20°C-50°C | [d] | [e] | Gruppe (2) | ja | 1:50-1:2.000 | [i] |
| 0,1-60 | 20°C-50°C | [d] | [e] | Gruppe (2) | nein | 1:50-1:2.000 | [i] |
| | | | | | | | |
| | | | | | | | |
| 0,1-200 | 0°C-120°C | [d] | [f] | Gruppe (1) | ja | 1:50-1:2.000 | [i] |
| 0,1-200 | 0°C-120°C | [d] | [f] | Gruppe (1) | nein | 1:50-1:2.000 | [i] |
| 0,1-200 | 10°C-100°C | [d] | [f] | Gruppe (1) | ja | 1:50-1:2.000 | [i] |
| 0,1-200 | 10°C-100°C | [d] | [f] | Gruppe (1) | nein | 1:50-1:2.000 | [i] |
| 0,1-200 | 20°C-50°C | [d] | [f] | Gruppe (1) | ja | 1:50-1:2.000 | [i] |
| 0,1-200 | 20°C-50°C | [d] | [f] | Gruppe (1) | nein | 1:50-1:2.000 | [i] |
| | | | | | | | |
| | | | | | | | |
| 0,1-100 | 0°C-120°C | [d] | [f] | Gruppe (1) | ja | 1:50-1:2.000 | [i] |
| 0,1-100 | 0°C-120°C | [d] | [f] | Gruppe (1) | nein | 1:50-1:2.000 | [i] |
| 0,1-100 | 10°C-100°C | [d] | [f] | Gruppe (1) | ja | 1:50-1:2.000 | [i] |
| 0,1-100 | 10°C-100°C | [d] | [f] | Gruppe (1) | nein | 1:50-1:2.000 | [i] |
| 0,1-100 | 20°C-50°C | [d] | [f] | Gruppe (1) | ja | 1:50-1:2.000 | [i] |
| 0,1-100 | 20°C-50°C | [d] | [f] | Gruppe (1) | nein | 1:50-1:2.000 | [i] |
| 0,1-60 | 0°C-120°C | [d] | [f] | Gruppe (1) | ja | 1:50-1:2.000 | [i] |
| 0,1-60 | 0°C-120°C | [d] | [f] | Gruppe (1) | nein | 1:50-1:2.000 | [i] |
| 0,1-60 | 10°C-100°C | [d] | [f] | Gruppe (1) | ja | 1:50-1:2.000 | [i] |
| 0,1-60 | 10°C-100°C | [d] | [f] | Gruppe (1) | nein | 1:50-1:2.000 | [i] |
| 0,1-60 | 20°C-50°C | [d] | [f] | Gruppe (1) | ja | 1:50-1:2.000 | [i] |
| 0,1-60 | 20°C-50°C | [d] | [f] | Gruppe (1) | nein | 1:50-1:2.000 | [i] |
| 0,1-200 | 0°C-120°C | [d] | [f] | Gruppe (2) | ja | 1:50-1:2.000 | [i] |
| 0,1-200 | 0°C-120°C | [d] | [f] | Gruppe (2) | nein | 1:50-1:2.000 | [i] |
| 0,1-200 | 10°C-100°C | [d] | [f] | Gruppe (2) | ja | 1:50-1:2.000 | [i] |
| 0,1-200 | 10°C-100°C | [d] | [f] | Gruppe (2) | nein | 1:50-1:2.000 | [i] |
| 0,1-200 | 20°C-50°C | [d] | [f] | Gruppe (2) | ja | 1:50-1:2.000 | [i] |
| 0,1-200 | 20°C-50°C | [d] | [f] | Gruppe (2) | nein | 1:50-1:2.000 | [i] |
| | | | | | | | |
| | | | | | | | |
| 0,1-100 | 0°C-120°C | [d] | [f] | Gruppe (2) | ja | 1:50-1:2.000 | [i] |
| 0,1-100 | 0°C-120°C | [d] | [f] | Gruppe (2) | nein | 1:50-1:2.000 | [i] |
| 0,1-100 | 10°C-100°C | [d] | [f] | Gruppe (2) | ja | 1:50-1:2.000 | [i] |
| 0,1-100 | 10°C-100°C | [d] | [f] | Gruppe (2) | nein | 1:50-1:2.000 | [i] |
| 0,1-100 | 20°C-50°C | [d] | [f] | Gruppe (2) | ja | 1:50-1:2.000 | [i] |
| 0,1-100 | 20°C-50°C | [d] | [f] | Gruppe (2) | nein | 1:50-1:2.000 | [i] |
| | | | | | | | |
| | | | | | | | |
| 0,1-60 | 0°C-120°C | [d] | [f] | Gruppe (2) | ja | 1:50-1:2.000 | [i] |
| 0,1-60 | 0°C-120°C | [d] | [f] | Gruppe (2) | nein | 1:50-1:2.000 | [i] |
| 0,1-60 | 10°C-100°C | [d] | [f] | Gruppe (2) | ja | 1:50-1:2.000 | [i] |
| 0,1-60 | 10°C-100°C | [d] | [f] | Gruppe (2) | nein | 1:50-1:2.000 | [i] |
| 0,1-60 | 20°C-50°C | [d] | [f] | Gruppe (2) | ja | 1:50-1:2.000 | [i] |
| 0,1-60 | 20°C-50°C | [d] | [f] | Gruppe (2) | nein | 1:50-1:2.000 | [i] |
| 40 bar H₂ | 20°C-50°C | [d] | [f] | K₂CO₃ ^{(a)}, NaOEt | ja | 1:20-1:10.000 | [i] |
| 40 bar H₂ | 20°-50°C | [d] | [f] | K₂CO₃ ^{(a)}, NaOEt | nein | 1:20-1:10.000 | [i] |
| 40 bar H₂ | 20°C-50°C | [d] | [f] | keine | ja | 1:20-1:10.000 | [i] |
| 40 bar H₂ | 20°-50°C | [d] | [f] | keine | nein | 1:20-1:10.000 | [i] |
| 40 bar H₂ | 20°C-50°C | [d] | [f] | K₂CO₃ ^{(a)}, NaOEt | ja | 1:50-1:2.000 | [i] |
| 40 bar H₂ | 20°-50°C | [d] | [f] | K₂CO₃ ^{(a)}, NaOEt | nein | 1:50-1:2.000 | [i] |
| 40 bar H₂ | 20°C-50°C | [d] | [f] | keine | ja | 1:50-1:2.000 | [i] |
| 40 bar H₂ | 20°-50°C | [d] | [f] | keine | nein | 1:50-1:2.000 | [i] |

| | | | | | | | |
|---|---|---|---|---|---|---|---|
| [a] = Zusatz eines Phasentransferkatalysators [b] = Hydrierkatalysator mit mindestens einem aktivem Metall der Gruppe VII B und/oder VIII B des Periodensystems der Elemente [c] = Pd/C, Pd/Al₂O₃, Pd/CaCO₃, Pt/C, Ru/Al₂O₃, Pd/Re/C, Pt/Re/C oder [(dppb)Rh(cod)]BF₄ [d] = 5%Pd/C, 5%Pd/Al₂O₃, 5%Pd/CaCO₃, 5%Pt/C, 5%Ru/Al₂O₃ oder [(dppb)Rh(cod)]BF₄ [e] = Alkylhalogenide, Ester der Sulfonsäure, Ester der Fluorsulfonsäure, Ester der Trifluormethansulfonsäure, Ester der Chlorameisensäure, Oxonium-Salze, Dialkylsulfate oder Diazomethan, [f] = (Me₃O)BF₄, (Et₃O)BF₄, (MeO)₂SO₂, (EtO)₂SO₂, ClCOOEt, MeI oder EtI [g] = Carbonsäureamid der allgemeinen Formel (I), Carbonsäurediamid der allgemeinen Formel (II), Di-, Tri- oder Polypeptid, Peptidamid mit carboxyerminaler Amidfunktion, [h] = Carbonsäureamid der allgemeinen Formel (I) [i] = Carbonsäureamide aus Tabelle 1 | | | | | | | |

Eine besondere Ausführungsform der Erfindung ist ein Verfahren zur Herstellung von Aminen, wobei das Alkylierungsmittel ausgewählt wird aus der Gruppe bestehend aus Alkylhalogeniden, Estern der Sulfonsäure, Estern der Fluorsulfonsäure, Estern der Trifluormethansulfonsäure, Estern der Chlorameisensäure, Oxonium-Salzen, Dialkylsulfaten und Diazomethan.

Eine weitere besondere Ausführungsform der Erfindung ist ein Verfahren zur Herstellung von Aminen, wobei nach Schritt a eine Base zum Reaktionsgemisch zugegeben wird und wobei ein Mol-Verhältnis von Base zu Alkylierungsmittel von 1:1 bis 1:3 gewählt wird.

Prinzipiell ist der Fachmann frei in der Wahl einer geeigneten basischen Verbindung. Vorzugsweise werden jedoch kostengünstige anorganische beziehungsweise organische Basen eingesetzt.
Die Base kann ausgewählt werden aus der Gruppe (1) bestehend aus Carbonaten, Hydrogencarbonaten, Phosphaten, Mono- oder Dihydrogenphosphaten oder Alkoxiden der Alkali- oder Erdalkalimetalle;
stickstoffhaltigen organischen Molekülen wie z. B. solche ausgewählt aus der Gruppe bestehend aus Triethylamin, Tri-n-butylamin, 1,4-Diazabicyclo[2,2,2]octan, 1,8-Diazabicyclo[5,4,0]undec-7-en, N,N-Dimethylglycinethylester, Pyridin, Tetramethylguanidin, N,N,N',N'-Tetramethylethan-1,2-diamin, Hexamethylentetramin; und
auf Oligomeren und Polymeren geträgerte Amine sowie deren Derivate.
Feste Basen werden bevorzugt in Gegenwart von einem Phasentransferkatalysator (PTC) eingesetzt. Beispiele für Phasentransferkatalysatoren sind die folgenden Verbindungen Dimethyl(di-(C₁₄-C₁₈)-alkyl)ammoniumchlorid, Methyl-tri-(C₈-C₁₀)-alkyl-ammoniumchlorid, Benzyl-dimethyl-stearylammoniumchlorid, Dimethyl-(C₁₂-C₁₆)-alkyl-benzylammonium-chlorid, Distearyl-dimethylammonium-chlorid (Coconut oil alkyl)bis(2-hydroxyethyl,ethoxylated)methylammonium-chlorid (= PEG-2-cocomonium-chlorid), Hexadecyl-(2-hydroxyethyl)-dimethylammonium dihydrogenphosphat, Trihexyl-tetradecylphosphonium chlorid, Trihexyl-tetradecylphosphonium bis(2,4,4-trimethylpentyl)phosphinat, N,N-Di(2-Tallowamidoethyl)-N-(2-hydroxyethyl)-N-methylammonium-methylsulfat, Di-(Oleyl-Carboxyethyl)-hydroxyethyl-methylammonium-methylsulfat, Di-(Palm-Carboxyethyl)-hydroxyethyl-methylammonium-methylsulfat, Bis(Soybean-amidoethyl)-polyethoxy-methylammonium-methylsulfat, 1-Methyl-2-nortallow-3-tallow-amidoethyl-imidazolinium-methylsulfat, 1-Ethyl-3-heptadecenyl-imidazolinium-methylsulfat, 1-Methyl-2-noroleyl-3-oleylalkyl-amidoethyl-imidazolinium-methylsulfat.
Bevorzugt sind solche Basen, die ausgewählt sind aus der Gruppe (2) bestehend aus K₃PO4, K₂HPO4, Ca₂CO₃, K₂CO₃, Na₂CO₃, NaOEt, NaOMe, NEt₃ und Pyridin, wobei diese Basen bevorzugt in Gegenwart eines Phasentransferkatalysators eingesetzt werden.

Die Menge der eingesetzten Base kann vom Fachmann frei gewählt werden, wobei ein Mol-Verhältnis von Base zu Alkylierungsmittel von 1:1 bis 1:3 bevorzugt ist. Besonders bevorzugt ist ein Mol-Verhältnis von 1:1.

Eine besondere Ausführungsform der Erfindung ist ein Verfahren zur Herstellung von Aminen, wobei die Umsetzung in einem Lösungsmittel durchgeführt wird.

Eine besondere Ausführungsform der Erfindung ist ein Verfahren zur Herstellung von Aminen, wobei das Lösungsmittel ausgewählt wird aus der Gruppe bestehend aus Kohlenwasserstoffen, Chlorkohlenwasserstoffen, Ethern, Estern und Alkoholen.

Eine weitere besondere Ausführungsform der Erfindung ist ein Verfahren zur Herstellung von Aminen, wobei die Umsetzung ohne Lösungsmittel durchgeführt wird.

Eine weitere besondere Ausführungsform der Erfindung ist ein Verfahren zur Herstellung von Aminen, wobei die Umsetzung ohne Lösungsmittel durchgeführt wird.

Es ist vorteilhaft wasserfrei zu arbeiten. Ein weiterer Gegenstand der Erfindung ist daher ein Verfahren, wobei wasserfreies Carbonsäureamid oder Carbonsäurediamid oder Di-, Tri- oder Polypeptid oder Peptidamid und wasserfreies Alkylierungsmittel und gegebenenfalls wasserfreies Lösungsmittel eingesetzt werden.

Bei dem erfindungsgemäßen Verfahren geht man im Allgemeinen so vor, dass man in einem Autoklaven das Carbonsäureamid oder Carbonsäurediamid oder Di-, Tri- oder Polypeptid oder Peptidamid und das Alkylierungsmittel miteinander reagieren lässt. Anschließen wird der Katalysator in einem bestimmten Mol-Verhältnis mit einer geeigneten Menge Lösungsmittel vermischt. Anschließend wird der Autoklav mehrmals mit Wasserstoff gespült und die Mischung bei geeignetem Druck hydriert. Nachdem der Wasserstoffdruck abgelassen wurde, wird die Reaktionsmischung abfiltriert und das Filtrat nach dem Fachmann bekannten Verfahren aufgearbeitet.
Bei einer bevorzugten Ausführungsform des erfindungsgemäßen Verfahrens geht man im Allgemeinen so vor, dass man in einem Autoklaven das Carbonsäureamid oder Carbonsäurediamid oder Di-, Tri- oder Polypeptid oder Peptidamid und das Alkylierungsmittel miteinander reagieren lässt. Anschließen wird mit einer geeigneten Menge Lösungsmittel und Base vermischt. Anschließend wird der Katalysator in einem bestimmten Mol-Verhältnis zugesetzt, der Autoklav wird mehrmals mit Wasserstoff gespült, und die Mischung wird bei geeignetem Druck hydriert. Nachdem der Wasserstoffdruck abgelassen wurde, wird die Reaktionsmischung abfiltriert und das Filtrat nach dem Fachmann bekannten Verfahren aufgearbeitet.

### Ausführungsbeispiele

Die nachstehenden Beispiele dienen zur Erläuterung der Erfindung, ohne sie darauf zu beschränken.

### BEISPIELE 1-4

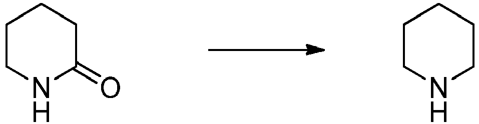

### Beispiel 1:

Piperidon-2 (5 g, 50 mmol) wird mit Chlorameisensäureethylester (6 ml, 60 mmol) versetzt und 4 Stunden bei 50°C unter Argon gerührt. Anschließend wird im Vakuum eingeengt, und der Rückstandwird in absolutem Ethanol aufgenommen. Ein Autoklav wird mit 5% Pt/C (0.98 g, 0.5mol%) befüllt, mit Argon gespült und mit Reaktionslösung in Ethanol gefüllt. Es werden 40 bar Wasserstoff aufgepresst, und es wird bei 25°C und konstantem Druck gerührt, bis keine Wasserstoffaufnahme mehr zu erkennen ist (5 h). Nach Abtrennen vom Katalysator wird das Filtrat am Rotationsverdampfer eingeengt, der Rückstand wird in 20 ml Wasser gelöst und mit Diethylether gewaschen, die wässrige Phase wird mit 2N NaOH-Lösung basisch gestellt und ausgeethert. Die organische Phase wird über K₂CO₃ getrocknet, nach Abdampfen des Ethers erhält man fast sauberes Piperidin. Die Ausbeute kann Tabelle 4 entnommen werden.

### Beispiel 2:

Piperidon-2 (5 g, 50 mmol) wird mit Dimethylsulfat (5 ml, 50 mmol) versetzt und 3 Stunden bei 80°C unter Argon gerührt, anschließend wird in 10 ml absolutem Methanol aufgenommen. Ein Autoklav wird mit 5% Pt/C (0.98 g, 0.5mol%) befüllt, mit Argon gespült und mit der Reaktionslösung in Methanol gefüllt. Es werden 40 bar Wasserstoff aufgepresst, und es wird bei 25°C und konstantem Druck gerührt, bis keine Wasserstoffaufnahme mehr zu erkennen ist (5 h). Nach Abtrennen vom Katalysator wird das Filtrat am Rotationsverdampfer eingeengt, der Rückstand wird in 20 ml Wasser gelöst und mit Diethylether gewaschen, die wässrige wird Phase mit 2N NaOH-Lösung basisch gestellt und ausgeethert. Die organische Phase wird über K₂CO₃ getrocknet, nach Abdampfen des Ethers erhält man fast sauberes Piperidin.

Die Ausbeute kann Tabelle 4 entnommen werden.

### Beispiel 3:

Piperidon-2 (5 g, 50 mmol)wird unter Feuchtigkeitausschluss und Kühlen auf einem Wasserbad mit Triethyloxonium-tetrafluoroborat (10.45 g, 55 mmol)versetzt. Dann wird die Wasserbadtemperatur allmählich auf 40°C erwärmt, und es wird bei dieser Temperatur 1 Std. gerührt. Anschließend wird der Ether im Vakuum abgezogen, und der Rückstandwird in absolutem Ethanol aufgenommen. Ein Autoklav wird mit 5% Pt/C (0.98 g, 0.5mol%) befüllt, mit Argon gespült und mit der Reaktionsmischung in Ethanol gefüllt. Es werden 40 bar Wasserstoff aufgepresst, und es wird bei 25°C und konstantem Druck gerührt, bis keine Wasserstoffaufnahme mehr zu erkennen ist (5 h). Nach Abtrennen vom Katalysator wird das Filtrat am Rotationsverdampfer eingeengt, der Rückstand wird in 20 ml Wasser gelöst und mit Diethylether gewaschen, die wässrige Phase wird mit 2N NaOH-Lösung basisch gestellt und ausgeethert. Die organische Phase wird über K₂CO₃ getrocknet, nach Abdampfen des Ethers erhält man fast sauberes Piperidin. Die Ausbeute kann Tabelle 4 entnommen werden.

### Beispiel 4:

Piperidon-2 (5 g, 50 mmol)wird unter Feuchtigkeitausschluss und Kühlen auf einem Wasserbad mit Triethyloxonium-tetrafluoroborat (10.45 g, 55 mmol)versetzt. Dann wird die Wasserbadtemperatur allmählich auf 40°C erwärmt, und es wird bei dieser Temperatur 1 Std. gerührt. Anschließend wird der Ether im Vakuum abgezogen, und der Rückstandwird in absolutem Ethanol aufgenommen. Ein Autoklav wird mit 5% Pt/C (0.98 g, 0.5mol%)und K₂CO₃ (6.9 g, 50 mmol) befüllt, mit Argon gespült und mit der Reaktionsmischung in Ethanol gefüllt. Es werden 40 bar Wasserstoff aufgepresst, und es wird bei 25°C und konstantem Druck gerührt, bis keine Wasserstoffaufnahme mehr zu erkennen ist (5 h). Nach Filtrieren über Celite wird das Filtrat in 25 ml 2N Salzsäure gelöst und mit Diethylether gewaschen, die wässrige Phase wird mit 2N NaOH-Lösung basisch gestellt und ausgeethert, die organische Phase wird über K₂CO₃ getrocknet. Nach Abdampfen des Ethers erhält man fast sauberes Piperidin.

Die Ausbeute kann Tabelle 4 entnommen werden.

**Tabelle 4: Umsetung von Piperidon-2 zu Piperidin.**

| Beispiel | Alkylierungsmittel | Base | Ausbeute, % |
|---|---|---|---|
| 1 | ClCOOEt | - | 80 |
| 2 | (MeO)₂SO₂ | - | 86 |
| 3 | (Et₃O)BF₄ | - | 85 |
| 4 | (Et₃O)BF₄ | K₂CO₃ | 86 |

### Beispiel 5:

Pyrrolidon-2 (4,25 g, 50 mmol) wird mit Chlorameisensäureethylester (6 ml, 60 mmol) versetzt und 4 Stunden bei 50°C unter Argon gerührt. Anschließend wird im Vakuum eingeengt, und der Rückstandwird in absolutem Ethanol aufgenommen. Ein Autoklav wird mit 5% Pt/C (0.98 g, 0.5 mol%) befüllt, mit Argon gespült und mit der Reaktionslösung in Ethanol gefüllt. Es werden 40 bar Wasserstoff aufgepresst, und es wird bei 25°C und konstantem Druck 24 Stunden gerührt. Nach Abtrennen vom Katalysator wird das Filtrat am Rotationsverdampfer eingeengt, der Rückstand wird in 20 ml Wasser gelöst und mit Diethylether gewaschen, die wässrige Phase wird mit 2N NaOH-Lösung basisch gestellt und ausgeethert. Die organische Phase wird über K₂CO₃ getrocknet, nach Abdampfen des Ethers erhält man fast sauberes Pyrrolidin. Ausbeute 1.85 g (52%).

### Beispiele 6-9:

Ein Carbonsäureamid (10 mmol) wird mit Dimethylsulfat (1 ml, 10 mmol) versetzt und 3 Stunden bei 80°C unter Argon gerührt, anschließend wird in 5 ml absolutem Methanol aufgenommen. Ein Autoklav wird mit Katalysator(1 mol%) und 5 ml 2M Natriummethylat-Lösung in Methanol befüllt, mit Argon gespült und mit der Reaktionslösung in Methanol gefüllt. Es werden 40 bar Wasserstoff aufgepresst, und es wird bei 25°C und konstantem Druck gerührt, bis keine Wasserstoffaufnahme mehr zu erkennen ist (1-2 h). Nach Filtrieren über Celite wird das Filtrat in 10 ml 2N Salzsäure gelöst und mit Diethylether gewaschen, die wässrige Phase wird mit 12 ml 2N NaOH-Lösung basisch gestellt und ausgeethert. Die kombinierten organischen Phasen werden über K₂CO₃ getrocknet und mit 10 ml 1M HCl-Lösung in Diethylether versetzt. Das feste Aminhydrochlorid wird abfiltriert, mit Diethylether gewaschen und im Vakuum getrocknet.

Die eingesetzten Katalysatoren und Ausbeuten können Tabelle 5 entnommen werden.

**Tabelle 5.**

| Beispiel | Katalysator | Carbonsäureamid | Produkt | Ausbeute[%] |
|---|---|---|---|---|
| 6 | 5%Pd/C | 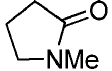 | 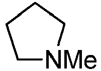 | 60 |
| 7 | 5%Ru/Al₂O₃ | 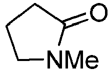 | 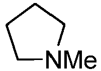 | 60 |
| 8 | 5%Pd/CaCO₃ | 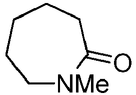 | 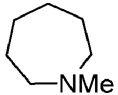 | 50 |
| 9 | [(dppb)Rh(cod)] BF₄ | 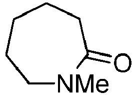 | 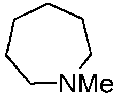 | 60 |

### Beispiele 10-28:

Eine Lösung von Carbonsäureamid (20 mmol) in Dichlormethan (10 ml) wird mit Triethyloxonium-tetrafluoroborat (4.18 g, 22 mmol) versetzt und entweder (A) über Nacht bei Raumtemperatur oder (B) 3 Stunden auf dem Wasserbad bei 40°C unter Argon gerührt. Anschließend wird im Vakuum eingeengt, und der Rückstand wird in 20 ml absolutem Ethanol aufgenommen. Ein im Eisbadgekühlter Autoklav wird mit Katalysator(1 mol%) und 10 ml 2M Natriumethylat-Lösung in Ethanol befüllt, mit Argon gespült und mit der Reaktionslösung in Ethanol gefüllt. Es werden 40 bar Wasserstoff aufgepresst, und es wird bei 25°C und konstantem Druck gerührt, bis keine Wasserstoffaufnahme mehr zu erkennen ist (1-12 h). Nach Filtrieren über Celite wird das Filtrat in 11 ml 2N Salzsäure gelöst und mit Diethylether gewaschen, die wässrige Phase wird mit 14 ml 2N NaOH-Lösung basisch gestellt, das Amin wird mit Diethylether extrahiert, und die kombinierten organischen Phasen werden über K₂CO₃ getrocknet. Nach Abziehen der Lösungsmittel im Vakuum erhält man praktisch sauberes Amin.

Die eingesetzten Katalysatoren und Ausbeuten können Tabelle 6 entnommen werden.

**Tabelle 6.**

| Beispiel | Katalysator | Edukt | Produkt | Ausbeute, % |
|---|---|---|---|---|
| 10 [A] | 5%Pd/CaCO₃ | 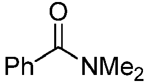 | 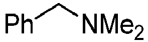 | 69 |
| 11 [A] | 5%Pt/C | 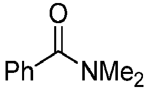 | 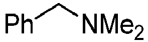 | 68 |
| 12 [A] | [(dppb)Rh(cod)]BF₄ | 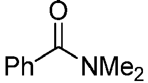 | 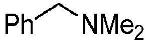 | 68 |
| 13 [A] | 5%Pd/Al₂O₃ | 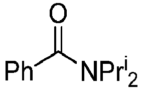 | 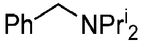 | 62 |
| 14 [A] | 5%Pt/C | 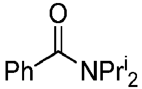 | 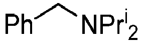 | 72 |
| 15 [B] | 5%Pd/C | 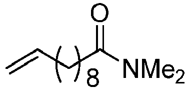 | 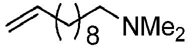 | 78 |
| 16 [B] | 5%Pd/CaCO₃ | 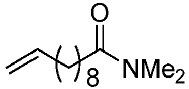 | 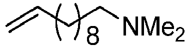 | 78 |
| 17 [B] | 5%Pd/Al₂O₃ | 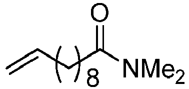 | 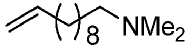 | 76 |
| 18 [B] | [(dppb)Rh(cod)]BF₄ | 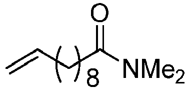 | 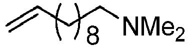 | 71 |
| 19 [B] | 5%Pd/C | 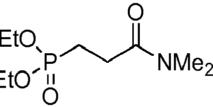 | 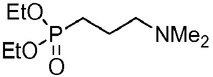 | 37 |
| 20 [B] | 5%Pt/C | 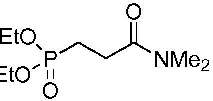 E | 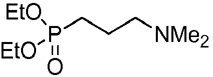 | 35 |
| 21 [B] | 5%Ru/Al₂O₃ | 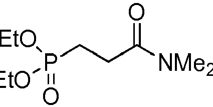 | 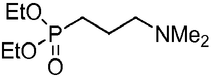 | 39 |
| 22 [B] | [(dppb)Rh(cod)]BF₄ | 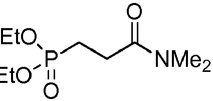 E | 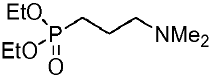 | 39 |
| 23 [B] | 5%Pd/C | 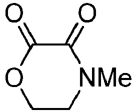 | 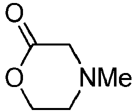 | 83 |
| 24 [B] | 5%Pd/CaCO₃ | 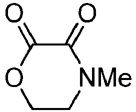 | 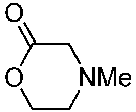 | 98 |
| 25 [B] | 5%Pd/Al₂O₃ | 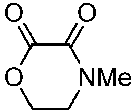 | 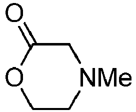 | 98 |
| 26 [B] | [(dppb)Rh(cod)] BF₄ | 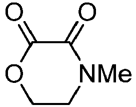 | 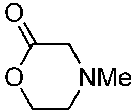 | 93 |
| 27 [A] | 5%Pd/C | 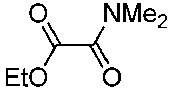 | 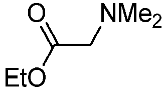 | 34 |
| 28 [A] | 5%Pt/C | 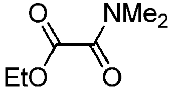 | 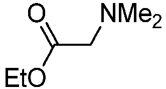 | 37 |

Bedingungen: [A] über Nacht bei Raumtemperatur gerührt, [B] 3 h bei 40°C gerührt.

### Beispiele 29-32:

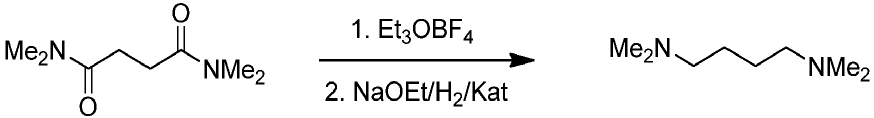

Eine Lösung von N,N'-Tetramethyl-bernsteinsäurediamid (20 mmol) in Dichlormethan(20 ml) wird mit Triethyloxonium-tetrafluoroborat (8.36 g, 44 mmol) versetzt und 3 Stunden auf dem Wasserbad bei 40°C unter Argon gerührt. Anschließend wird im Vakuum eingeengt, und der Rückstandwird in 50 ml absolutem Ethanol aufgenommen. Ein im Eisbadgekühlter Autoklav wird mit Katalysator(1 mol%) und 20 ml 2M Natriumethylat-Lösung in Ethanol befüllt, mit Argon gespült und mit der Reaktionsmischung in Ethanol gefüllt. Es werden 40 bar Wasserstoff aufgepresst, und es wird bei 25°C und konstantem Druck gerührt, bis keine Wasserstoffaufnahme mehr zu erkennen ist (1-12 h). Nach Filtrieren über Celite wird das Filtrat wie in den Beispielen 10-28 aufgearbeitet.

Die eingesetzten Katalysatoren und Ausbeuten an N,N'-Tetramethyl-1,4-butandiamin können Tabelle 7 entnommen werden.

**Tabelle 7: Umsetzung von N,N'-Tetramethyl-bernsteinsäurediamid zu N,N'-Tetramethyl-1,4-butandiamin**

| Beispiel | Katalysator | Ausbeute, % |
|---|---|---|
| 29 | 5%Pd/C | 60 |
| 30 | 5%Pd/Al₂O₃ | 62 |
| 31 | 5%Ru/Al₂O₃ | 63 |
| 32 | [(dppb)Rh(cod)]BF₄ | 67 |

### Beispiel 33:

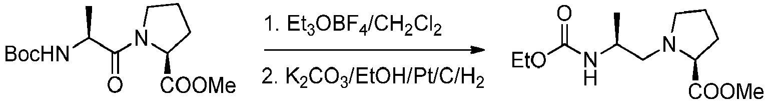

Eine Lösung von N-Boc-Ala-Pro-OMe (3.0 g, 10 mmol) in Dichlormethan (10 ml) wird mit Triethyloxonium-tetrafluoroborat (3.80 g, 20 mmol) versetzt und über Nacht bei Raumtemperaturunter Argon gerührt. Anschließend wird im Vakuum eingeengt, und der Rückstand wird in 20 ml absolutem Ethanol aufgenommen. Ein Autoklav wird mit 5%Pt/C(0.78 g, 2mol%), Trioctylmethylammonium-chlorid (Aliquat 336, 40 mg, 0.1 mmol) und K₂CO₃ (2.76 g, 20 mmol) befühlt, mit Argon gespült und mit der Reaktionsmischung in Ethanol gefüllt. Es werden 40 bar Wasserstoff aufgepresst, und es wird bei 25°C und konstantem Druck 16 Stunden gerührt. Nach Filtrieren über Celite wird das Filtrat im Vakuum eingeengt, und der Rückstand wird durch Säulenchromatographie (Essigsäure-ethylester/Hexan 1:1)gereinigt. Es werden 1.42 g (55%) (2'*S*,2*S*)*-*1*-*(2-Ethoxycarbonylamino-propyl)-pyrrolidin-2-carbonsäuremethylester (Rf 0.25) erhalten.
¹H NMR (CDCl₃) δ5.19 (br. s, 1H), 3.99-4.07 (m, 2H), 3.63 (s, 3H), 3.60 (q, J=6.4, 1H), 3.23 (dd, J = 8.6, J = 5.1, 1H), 3.09 (ddd, J = 8.3, J = 8.2, J = 3.9, 1H), 2.60 (dd, J = 12.4, J = 6.0, 1H), 2.50 (dd, J = 11.7, J = 5.9, 1H), 2.47 (dt, J = 8.9, J = 7.5, 1H), 2.00-2.05 (m, 1H), 1.81-1.86 (m, 2H), 1.72-1.78 (m, 1H), 1.17 (t, J = 7.1, 3H), 1.13 (d, J = 6.6, 3H); ¹³C δ 174.80, 156.28, 66.50, 60.38, 60.20, 54.44, 51.69, 46.41, 29.39, 23.83, 19.48, 14.64.

### Beispiel 34:

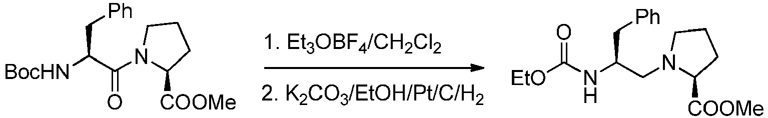

Eine Lösung von N-Boc-Phe-Pro-OMe (3.87 g, 10 mmol) in Dichlormethan (10 ml) wird mit Triethyloxonium-tetrafluoroborat (3.80 g, 20 mmol) versetzt und über Nacht bei Raumtemperatur unter Argon gerührt. Anschließend wird im Vakuum eingeengt, und der Rückstand wird in 20 ml absoluten Ethanol aufgenommen. Ein Autoklav wird mit 5%Pt/C(0.78 g, 2 mol%), Trioctylmethylammonium-chlorid (Aliquat 336, 40 mg, 0.1 mmol) und K₂CO₃ (2.76 g, 20 mmol) befüllt, mit Argon gespült und mit der Reaktionsmischung in Ethanol gefüllt. Es werden40 bar Wasserstoff aufgepresst, und es wird bei 25°C und konstantem Druck 16 Stunden gerührt. Nach Filtrieren über Celite wird das Filtrat im Vakuum eingeengt, und der Rückstand wird durch Säulenchromatographie (Essigsäure-ethylester/Hexan 1:1) gereinigt. Es werden 1.18 g (34%) (2'S,2S)-1-(2-Ethoxycarbonylamino-3-phenyl-propyl)-pyrrolidin-2-carbonsäuremethylester (Rf 0.40) erhalten.
¹H NMR (CDCl₃) δ7.20 (t, J = 7.6, 2H), 7.12 (t, J = 7.8, 3H), 5.46 (br. s, 1H), 4.07 (dq, J = 10.6, J = 7.2, 1H), 4.02 (dq, J = 10.6, J = 7.1, 1H), 3.75 (br. m, 1H), 3.61 (s, 3H), 3.18 (br. m, 1H), 3.07 (br. m, 1H), 3.03 (td, J = 7.9, J = 3.4, 1H),2.58 (dd, J = 13.5, J = 7.8, 1H), 2.52 (t, J = 11.3, 1H), 2.40 (dd, J = 12.1, J = 3.85, 1H), 2.21 (br. m, 1H), 1.98-2.04 (m, 1H), 1.98-2.04 (m, 1H), 1.66-1.72 (m, 2H), 1.18 (t, J = 7.2, 3H); ¹³C δ 174.71, 156.84, 138.20, 129.48, 128.29, 126.25, 65.51, 60.54, 56.79, 53.04, 51.81, 51.44, 39.48 , 29.21, 23.43, 14.69.

### Beispiel 35:

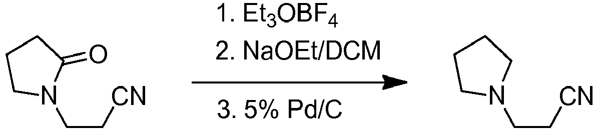

In einem im Eisbad gekühlten Schlenk-Gefäß wird N-(2-Cyanoethyl)-pyrrolidon-2 (6.9 g, 50 mmol) mit Triethyloxoniumtetrafluoroborat (10.45 g, 55 mmol) versetzt und über Nacht bei Raumtemperatur unter Argon gerührt. Die Ether-Phase wird abdekantiert und noch enthaltener Ether wird im Vakuum entfernt. Der Rückstand wird in trockenem Dichlormethan (40 ml) gelöst und zu einer Suspension von Natrium-ethoxid (ethanolfrei) in 10 ml trockenem Diethylether bei -20°C zugetropft. Anschließend lässt man über Nacht auf Raumtemperatur unter kräftigem Rühren aufwärmen und filtriert unter Argon vom NaBF₄ ab. Das Filtrat wird eingeengt und in 10 ml trockenem Dichlormethan gelöst. Ein Autoklav wird mit 5% Pt/C (390 mg, 0.2mol%) befüllt, mit Argon gespült und mit der Reaktionslösung in Dichlormethan gefüllt. Es werden 40 bar Wasserstoff aufgepresst, und es wird bei 25°C und konstantem Druck 24 Stunden gerührt. Nach Filtrieren über Celite wird das Filtrat in 40 ml eiskalter 2N Salzsäure gelöst und mit Diethylether gewaschen, die wässrige Phase wird unter Eiskühlung mit 42 ml 2N NaOH-Lösung basisch gestellt, das Produkt wird mit Diethylether extrahiert (6 x 20 ml), und die kombinierten organischen Phasen werden über K₂CO₃ getrocknet. Nach Abziehen der Lösungsmittel im Vakuum erhält man 2.6 g (44%) praktisch sauberen 3-(1-Pyrrolidino)-propionitril.

## Patentansprüche

1. Verfahren zur Herstellung von Aminen umfassend die folgenden Schritte:
a. Umsetzung eines
i. Carbonsäureamids der allgemeinen Formel (I) 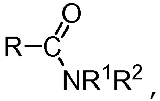 wobei
R ausgewählt wird aus der Gruppe bestehend aus H, substituiertes oder unsubstituiertes (C₁-C₂₄)-Alkyl, substituiertes oder unsubstituiertes (C₃-C₂₀)-Cycloalkyl, substituiertes oder unsubstituiertes (C₂-C₁₃)-Heterocycloalkyl, substituiertes oder unsubstituiertes (C₆-C₁₄)-Aryl, substituiertes oder unsubstituiertes (C₃-C₁₃)-Heteroaryl und dem Säurerest einer Aminosäure ausgewählt aus Alanin, Arginin, Asparagin, Asparaginsäure, Cystein, Glutamin, Glutaminsäure, Glycin, Histidin, Isoleucin, Leucin, Lysin, Methionin, Phenylalanin, Prolin, Serin, Threonin, Tryptophan, Tyrosin, Valin; und
R¹ und R² unabhängig voneinander ausgewählt werden aus der Gruppe bestehend aus H, substituiertes oder unsubstituiertes (C₁-C₂₄)-Alkyl, substituiertes oder unsubstituiertes (C₃-C₈)-Cycloalkyl, substituiertes oder unsubstituiertes (C₂-C₇)-Heterocycloalkyl, substituiertes oder unsubstituiertes (C₆-C₁₄)-Aryl oder substituiertes oder unsubstituiertes (C₃-C₁₃)-Heteroaryl, wobei sowohl die Reste R mit R¹, R mit R² als auch R¹ mit R² unabhängig voneinander eine gesättigte oder einfach oder mehrfach ungesättigte (C₂-C₁₈)-Alkylen-oder (C₂-C₁₈)-Heteroalkylen-Brücke bilden, so dass ein aliphatischer oder aromatischer Ring mit insgesamt 3-20 Ringatomen entsteht,
oder
ii. Carbonsäurediamids der allgemeinen Formel (II) 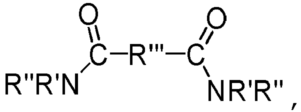 wobei
R"' ausgewählt wird aus der Gruppe bestehend aus divalenten substituierten oder unsubstituierten (C₁-C₂₄)-Alkylresten, substituierten oder unsubstituierten (C₃-C₂₀)-Cycloalkylresten, substituierten oder unsubstituierten (C₂-C₁₃)-Heterocycloalkylresten, substituierten oder unsubstituierten (C₆-C₁₄)-Arylresten, substituierten oder unsubstituierten (C₃-C₁₃)-Heteroarylresten; und
R' und R" unabhängig voneinander ausgewählt werden aus der Gruppe bestehend aus H, substituiertes oder unsubstituiertes (C₁-C₂₄)-Alkyl, substituiertes oder unsubstituiertes (C₁-C₂₄)-Heteroalkyl, substituiertes oder unsubstituiertes (C₃-C₈)-Cycloalkyl, substituiertes oder unsubstituiertes (C₂-C₇)-Heterocycloalkyl, substituiertes oder unsubstituiertes (C₆-C₁₄)-Aryl oder substituiertes oder unsubstituiertes (C₃-C₁₃)-Heteroaryl, wobei die Reste R' mit R" miteinander eine gesättigte oder einfach oder mehrfach ungesättigte (C₂-C₁₈)-Alkylen- oder (C₂-C₁₈)-Heteroalkylen-Brücke bilden können, so dass ein aliphatischer oder aromatischer Ring mit insgesamt 3-20 Ringatomen entsteht,
oder
iii. Di-, Tri- oder Polypeptids, oder
iv. Peptidamids mit carboxyterminaler Amidfunktion mit einem Alkylierungsmittel,
b. Zugabe eines Hydrierkatalysators, der mindestens ein aktives Metall der Gruppe VII B und/oder VIII B des Periodensystems der Elemente enthält, zum Reaktionsgemisch, wobei das Mol-Verhältnis von Hydrierkatalysator zu Carbonsäureamid oder Carbonsäurediamid oder Di-, Tri- oder Polypeptid oder Peptidamid in einem Bereich von 1:10 bis 1:100.000 liegt,
c. Umsetzung des Reaktionsgemisches mit Wasserstoff, wobei ein Wasserstoffdruck von 0,1 bar bis 200 bar eingestellt wird und wobei eine Temperatur im Bereich von 0°C bis 120°C eingestellt wird.

2. Verfahren gemäß Anspruch 1, wobei das Alkylierungsmittel ausgewählt wird aus der Gruppe bestehend aus Alkylhalogeniden, Estern der Sulfonsäure, Estern der Fluorsulfonsäure, Estern der Trifluormethansulfonsäure, Estern der Chlorameisensäure, Oxonium-Salzen, Dialkylsulfaten und Diazomethan.

3. Verfahren gemäß einem der Ansprüche 1-2, wobei nach Schritt a eine Base zum Reaktionsgemisch zugegeben wird und wobei ein Mol-Verhältnis von Base zu Alkylierungsmittel von 1:1 bis 1:3 gewählt wird.

4. Verfahren gemäß einem der Ansprüche 1-3, wobei die Umsetzung in einem Lösungsmittel durchgeführt wird.

5. Verfahren gemäß Anspruch 4, wobei das Lösungsmittel ausgewählt wird aus der Gruppe bestehend aus Kohlenwasserstoffen, Chlorkohlenwasserstoffen, Ethern, Estern und Alkoholen.

6. Verfahren gemäß einem der Ansprüche 1-3, wobei die Umsetzung ohne Lösungsmittel durchgeführt wird.

7. Verfahren gemäß Anspruch 4 oder 5, wobei wasserfreies Carbonsäureamid oder Carbonsäurediamid oder Di-, Tri- oder Polypeptid oder Peptidamid, wasserfreies Alkylierungsmittel und wasserfreies Lösungsmittel eingesetzt werden.

8. Verfahren gemäß Anspruch 6, wobei wasserfreies Carbonsäureamid oder Carbonsäurediamid oder Di-, Tri- oder Polypeptid oder Peptidamid und wasserfreies Alkylierungsmittel eingesetzt werden.

9. Verfahren gemäß einem der Ansprüche 1-8, wobei alle (C₁-Cₙ)-Alkyl-Reste, (C₁-Cₙ)-Heteroalkyl-Reste, (C₃-Cₙ)-Cycloalkyl-Reste, (C₂-Cₙ)-Heterocycloalkyl-Reste, (C₆-Cₙ)-Aryl-Reste, und (C₃-Cₙ)-Heteroaryl-Reste unsubstituiert sind.

## Claims

1. Process for the preparation of amines comprising the following steps:
a. reaction of a
i. carboxylic acid amide of the general formula (I) 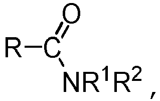 where
R is selected from the group consisting of H, substituted or unsubstituted (C₁-C₂₄)-alkyl, substituted or unsubstituted (C₃-C₂₀)-cycloalkyl, substituted or unsubstituted (C₂-C₁₃)-heterocycloalkyl, substituted or unsubstituted (C₆-C₁₄)-aryl, substituted or unsubstituted (C₃-C₁₃)-heteroaryl and the acid radical of an amino acid selected from alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, valine; and
R¹ and R², independently of one another, are selected from the group consisting of H, substituted or unsubstituted (C₁-C₂₄)-alkyl, substituted or unsubstituted (C₃-C₈)-cycloalkyl, substituted or unsubstituted (C₂-C₇)-heterocycloalkyl, substituted or unsubstituted (C₆-C₁₄)-aryl or substituted or unsubstituted (C₃-C₁₃)-heteroaryl, where both the radicals R with R¹, R with R² and also R¹ with R², independently of one another, form a saturated or mono- or polyunsaturated (C₂-C₁₈)-alkylene or (C₂-C₁₈)-heteroalkylene bridge, such that an aliphatic or aromatic ring having in total 3-20 ring atoms is formed,
or
ii. carboxylic acid diamide of the general formula (II) 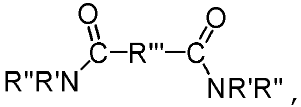 where
R"' is selected from the group consisting of divalent substituted or unsubstituted (C₁-C₂₄)-alkyl radicals, substituted or unsubstituted (C₃-C₂₀)-cycloalkyl radicals, substituted or unsubstituted (C₂-C₁₃)-heterocycloalkyl radicals, substituted or unsubstituted (C₆-C₁₄)-aryl radicals, substituted or unsubstituted (C₃-C₁₃)-heteroaryl radicals; and
R' and R", independently of one another, are selected from the group consisting of H, substituted or unsubstituted (C₁-C₂₄)-alkyl, substituted or unsubstituted (C₁-C₂₄)-heteroalkyl, substituted or unsubstituted (C₃-C₈)-cycloalkyl, substituted or unsubstituted (C₂-C₇)-heterocycloalkyl, substituted or unsubstituted (C₆-C₁₄)-aryl or substituted or unsubstituted (C₃-C₁₃)-heteroaryl, where the radicals R' with R" can together form a saturated or mono- or polyunsaturated (C₂-C₁₈)-alkylene or (C₂-C₁₈)-heteroalkylene bridge, such that an aliphatic or aromatic ring having in total 3-20 ring atoms is formed,
or
iii. di-, tri- or polypeptide, or
iv. peptide amide with carboxy-terminal amide function
with an alkylating agent,
b. addition of a hydrogenation catalyst, which comprises at least one active metal of group VII B and/or VIII B of the Periodic Table of the Elements, to the reaction mixture, where the molar ratio of hydrogenation catalyst to carboxylic acid amide or carboxylic acid diamide or di-, tri- or polypeptide or peptide amide is in a range of from 1:10 to 1:100 000,
c. reaction of the reaction mixture with hydrogen, where a hydrogen pressure of from 0.1 bar to 200 bar is established and where a temperature in the range of from 0°C to 120°C is established.

2. Process according to Claim 1, where the alkylating agent is selected from the group consisting of alkyl halides, esters of sulphonic acid, esters of fluorosulphonic acid, esters of trifluoromethanesulphonic acid, esters of chloroformic acid, oxonium salts, dialkyl sulphates and diazomethane.

3. Process according to one of Claims 1-2, where, after step a, a base is added to the reaction mixture and where a molar ratio of base to alkylating agent of from 1:1 to 1:3 is chosen.

4. Process according to one of Claims 1-3, where the reaction is carried out in a solvent.

5. Process according to Claim 4, where the solvent is selected from the group consisting of hydrocarbons, chlorinated hydrocarbons, ethers, esters and alcohols.

6. Process according to one of Claims 1-3, where the reaction is carried out without solvents.

7. Process according to Claim 4 or 5, where anhydrous carboxylic acid amide or carboxylic acid diamide or di-, tri- or polypeptide or peptide amide, anhydrous alkylating agent and anhydrous solvent are used.

8. Process according to Claim 6, where anhydrous carboxylic acid amide or carboxylic acid diamide or di-, tri- or polypeptide or peptide amide and anhydrous alkylating agent are used.

9. Process according to one of Claims 1-8, where all (C₁-Cₙ)-alkyl radicals, (C₁-Cₙ)-heteroalkyl radicals, (C₃-Cₙ)-cycloalkyl radicals, (C₂-Cₙ)-heterocycloalkyl radicals, (C₆-Cₙ)-aryl radicals, and (C₃-Cₙ)-heteroaryl radicals are unsubstituted.

## Revendications

1. Procédé pour la préparation d'amines, comprenant les étapes suivantes :
a. transformation
i. d'un amide d'acide carboxylique de formule générale (I) 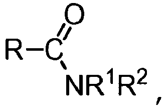 dans laquelle
R est choisi dans le groupe constitué par H, (C₁-C₂₄)-alkyle substitué ou non substitué, (C₃-C₂₀)-cycloalkyle substitué ou non substitué, (C₂-C₁₃)-hétérocycloalkyle substitué ou non substitué, (C₆-C₁₄)-aryle substitué ou non substitué ou (C₃-C₁₃)-hétéroaryle substitué ou non substitué et le radical acide est un acide aminé choisi parmi l'alanine, l'arginine, l'asparagine, l'acide aspartique, la cystéine, la glutamine, l'acide glutamique, la glycine, l'histidine, l'isoleucine, la leucine, la lysine, la méthionine, la phénylalanine, la proline, la sérine, la thréonine, le tryptophane, la tyrosine, la valine ; et
R¹ et R² sont choisis, indépendamment l'un de l'autre, dans le groupe constitué par H, (C₁-C₂₄)-alkyle substitué ou non substitué, (C₃-C₈)-cycloalkyle substitué ou non substitué, (C₂-C₇)-hétérocycloalkyle substitué ou non substitué, (C₆-C₁₄)-aryle substitué ou non substitué ou (C₃-C₁₃)-hétéroaryle substitué ou non substitué, le radical R et R¹, le radical R et R² et les radicaux R¹ et R² formant, indépendamment les uns des autres, un pont (C₂-C₁₈)-alkylène ou (C₂-C₁₈)-hétéroalkylène saturé ou monoinsaturé ou polyinsaturé, de telle sorte qu'il se forme un cycle aliphatique ou aromatique comprenant au total 3-20 atomes de cycle, ou
ii. d'un diamide d'acide carboxylique de formule générale (II) 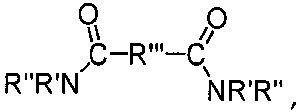 dans laquelle
R"' est choisi dans le groupe constitué par les radicaux (C₁-C₂₄)-alkyle substitués ou non substitués, les radicaux (C₃-C₂₀)-cycloalkyle substitués ou non substitués, les radicaux (C₂-C₁₃)-hétérocycloalkyle substitués ou non substitués, les radicaux (C₆-C₁₄)-aryle substitués ou non substitués, les radicaux (C₃-C₁₃)-hétéroaryle substitués ou non substitués divalents ; et
R' et R" sont choisis, indépendamment l'un de l'autre, dans le groupe constitué par H, (C₁-C₂₄)-alkyle substitué ou non substitué, (C₁-C₂₄)-hétéroalkyle substitué ou non substitué, (C₃-C₈)-cycloalkyle substitué ou non substitué, (C₂-C₇)-hétérocycloalkyle substitué ou non substitué, (C₆-C₁₄)-aryle substitué ou non substitué ou (C₃-C₁₃)-hétéroaryle substitué ou non substitué, les radicaux R' et R" pouvant former l'un avec l'autre un pont (C₂-C₁₈)-alkylène ou (C₂-C₁₈)-hétéroalkylène saturé ou monoinsaturé ou polyinsaturé, de telle sorte qu'il se forme un cycle aliphatique ou aromatique comprenant au total 3-20 atomes de cycle, ou
iii. d'un dipeptide, d'un tripeptide ou d'un polypeptide ou
iv. d'un peptidamide présentant une fonction amide en l'extrémité carboxy
avec un agent d'alkylation,
b. addition d'un catalyseur d'hydrogénation, qui contient au moins un métal actif du groupe VII B et/ou VIII B du système périodique des éléments, au mélange réactionnel, le rapport molaire de catalyseur d'hydrogénation à amide d'acide carboxylique ou à diamide d'acide carboxylique ou à dipeptide, tripeptide ou polypeptide ou à peptidamide se situant dans une plage de 1:10 à 1:100.000,
c. transformation du mélange réactionnel avec de l'hydrogène, une pression d'hydrogène de 0,1 à 200 bars et une température dans la plage de 0°C à 120°C étant réglées.

2. Procédé selon la revendication 1, l'agent d'alkylation étant choisi dans le groupe constitué par les halogénures d'alkyle, les esters de l'acide sulfonique, les esters de l'acide fluorosulfonique, les esters de l'acide trifluorométhanesulfonique, les esters de l'acide chloroformique, les sels d'oxonium, les sulfates de dialkyle et le diazométhane.

3. Procédé selon l'une quelconque des revendications 1-2, une base étant ajoutée au mélange réactionnel après l'étape a et un rapport molaire de base à agent d'alkylation de 1:1 à 1:3 étant choisi.

4. Procédé selon l'une quelconque des revendications 1-3, la transformation étant réalisée dans un solvant.

5. Procédé selon la revendication 4, le solvant étant choisi dans le groupe constitué par les hydrocarbures, les hydrocarbures chlorés, les éthers, les esters et les alcools.

6. Procédé selon l'une quelconque des revendications 1-3, la transformation étant réalisée sans solvant.

7. Procédé selon la revendication 4 ou 5, de l'amide d'acide carboxylique anhydre ou du diamide d'acide carboxylique anhydre ou du dipeptide, du tripeptide ou du polypeptide anhydre, du peptidamide anhydre, de l'agent d'alkylation anhydre et du solvant anhydre étant utilisés.

8. Procédé selon la revendication 6, de l'amide d'acide carboxylique anhydre ou du diamide d'acide carboxylique anhydre ou du dipeptide, du tripeptide ou du polypeptide anhydre ou du peptidamide anhydre et de l'agent d'alkylation anhydre étant utilisés.

9. Procédé selon l'une quelconque des revendications 1-8, les radicaux (C₁-Cₙ)-alkyle, (C₁-Cₙ)-hétéroalkyle, (C₃-Cₙ)-cycloalkyle, (C₂-Cₙ)-hétérocycloalkyle, (C₆-Cₙ)-aryle et (C₃-Cₙ)-hétéroaryle étant tous non substitués.
